# EUROPEAN PATENT APPLICATION

(11) **EP 3 524 675 A1**
(43) Date of publication of application: **14.08.2019**
(21) Application number: 18156413.9
(22) Date of filing: 13.02.2018
(51) Int. Cl.: C12N 9/88, C12P 17/02

(54) **PREPARATION OF TERTIARY ALCOHOLS, RESOLUTION OF TERTIARY ALCOHOLS AND STEREOSELECTIVE DEUTERATION OR TRITIATION BY RETROALDOLASES**

(71) Applicant: ETH Zürich, 8092 Zürich (CH)
(72) Inventor: Hilvert, Donald Michael, 8006 Zurich (CH); Garrabou Pi, Xavier, 8706 Meilen (CH); Macdonald, Duncan Stuart, 8032 Zurich (CH)
(74) Representative: Kasche, André

(57) **Abstract**

The present invention is directed to methods for catalyzing a chemical reaction by retroaldolases, corresponding uses of retroaldolases and to novel retroaldolases. The methods and retroaldolases have utility in (i) preparing tertiary alcohols, in (ii) chiral resolution of tertiary alcohols by retroaldol cleavage, and in (iii) deuteration or tritiation of carbonyl compounds.

SEQUENCE LISTING

<110>  ETH Zurich

<120>  Preparation of tertiary alcohols, resolution of tertiary alcohols
        and stereoselective deuteration or tritiation by retroaldolases

<130>  2017-094

<160>  53

<170>  PatentIn version 3.5

<210>  1
<211>  258
<212>  PRT
<213>  Artificial Sequence

<220>
<223>  Retroaldolase RA95.5-8F

<400>  1

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  2
<211>  258
<212>  PRT
<213>  Artificial Sequence

<220>
<223>  Retroaldolase 95.5-8

<400>  2

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Thr Arg Lys Ser Pro Ser Gly Leu Asp Val Glu Arg
    50                  55                  60

Asp Pro Ile Glu Tyr Ala Lys Tyr Met Glu Arg Tyr Ala Val Gly Leu
65                  70                  75                  80

Ser Ile Lys Thr Glu Glu Lys Tyr Phe Asn Gly Ser Tyr Glu Met Leu
                85                  90                  95

Arg Lys Ile Ala Ser Ser Val Ser Ile Pro Ile Leu Met Asn Asp Phe

100                 105                 110

Ile Val Lys Glu Ser Gln Ile Asp Asp Ala Tyr Asn Leu Gly Ala Asp
        115                 120                 125

Thr Val Leu Leu Ile Val Asn Ile Leu Thr Glu Arg Glu Leu Glu Ser
    130                 135                 140

Leu Leu Glu Tyr Ala Arg Ser Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Val Ile Phe Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Ala Lys Leu Asp Ile Ser Glu Arg Asn Glu Ile Glu Glu Leu Arg Lys
    210                 215                 220

Leu Gly Val Asn Ala Phe Leu Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  3
<211>  244
<212>  PRT
<213>  Artificial Sequence

<220>
<223>  Retroaldolase RA9558F shortened

<400>  3

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  4
<211>  244
<212>  PRT
<213>  Artificial Sequence

<220>
<223>  Retroaldolase RA9558 shortened

<400>  4

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Thr Arg Lys Ser Pro Ser Gly Leu Asp Val Glu Arg Asp
    50                  55                  60

Pro Ile Glu Tyr Ala Lys Tyr Met Glu Arg Tyr Ala Val Gly Leu Ser
65                  70                  75                  80

Ile Lys Thr Glu Glu Lys Tyr Phe Asn Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Asn Ile Leu Thr Glu Arg Glu Leu Glu Ser Leu
    130                 135                 140

Leu Glu Tyr Ala Arg Ser Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Val Ile Phe Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Ala
        195                 200                 205

Lys Leu Asp Ile Ser Glu Arg Asn Glu Ile Glu Glu Leu Arg Lys Leu
    210                 215                 220

Gly Val Asn Ala Phe Leu Ile Ser Ser Ser Leu Met Arg Asn Pro Glu
225                 230                 235                 240

Lys Ile Lys Glu

<210>  5
<211>  244
<212>  PRT
<213>  Artificial Sequence

<220>
<223>  Retroaldolase V12G RA9558F shortened

<400>  5

Pro Arg Tyr Leu Lys Gly Trp Leu Glu Asp Gly Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  6
<211>  244
<212>  PRT
<213>  Artificial Sequence

<220>
<223>  Retroaldolase V12A RA9558F shortened

<400>  6

Pro Arg Tyr Leu Lys Gly Trp Leu Glu Asp Ala Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  7
<211>  244
<212>  PRT
<213>  Artificial Sequence

<220>
<223>  Retroaldolase V12F RA9558F shortened

<400>  7

Pro Arg Tyr Leu Lys Gly Trp Leu Glu Asp Phe Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr

115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  8
<211>  244
<212>  PRT
<213>  Artificial Sequence

<220>
<223>  Retroaldolase F112I RA9558F shortened

<400>  8

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  9
<211>  244
<212>  PRT
<213>  Artificial Sequence

<220>
<223>  Retroaldolase F112L RA9558F shortened

<400>  9

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Leu Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  10
<211>  244
<212>  PRT
<213>  Artificial Sequence

<220>

<223>  Retroaldolase F112V RA9558F shortened

<400>  10

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Val Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210> 11
<211> 244
<212> PRT
<213> Artificial Sequence

<220>
<223> Retroaldolase V12F F112I RA9558F shortened

<400> 11

Pro Arg Tyr Leu Lys Gly Trp Leu Glu Asp Phe Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210> 12
<211> 244
<212> PRT
<213> Artificial Sequence

<220>
<223> Retroaldolase V12F F112L RA9558F shortened

<400> 12

Pro Arg Tyr Leu Lys Gly Trp Leu Glu Asp Phe Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Leu Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn

145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  13
<211>  244
<212>  PRT
<213>  Artificial Sequence

<220>
<223>  Retroaldolase V12G F112I RA9558F shortened

<400>  13

Pro Arg Tyr Leu Lys Gly Trp Leu Glu Asp Gly Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  14
<211>  244
<212>  PRT
<213>  Artificial Sequence

<220>
<223>  Retroaldolase V12A F112V RA9558F shortened

<400>  14

Pro Arg Tyr Leu Lys Gly Trp Leu Glu Asp Ala Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Val Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  15
<211>  244
<212>  PRT
<213>  Artificial Sequence

<220>
<223>  Retroaldolase V12A F112L RA9558F shortened

<400>  15

Pro Arg Tyr Leu Lys Gly Trp Leu Glu Asp Ala Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Leu Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  16
<211>  244
<212>  PRT

<213>  Artificial Sequence

<220>
<223>  Retroaldolase V12L F112V RA9558F shortened

<400>  16

Pro Arg Tyr Leu Lys Gly Trp Leu Glu Asp Leu Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Val Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  17
<211>  244
<212>  PRT
<213>  Artificial Sequence

<220>
<223>  Retroaldolase V12W F112V RA9558F shortened

<400>  17

Pro Arg Tyr Leu Lys Gly Trp Leu Glu Asp Trp Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Val Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn

180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  18
<211>  244
<212>  PRT
<213>  Artificial Sequence

<220>
<223>  Retroaldolase V12W F112I RA9558F shortened

<400>  18

Pro Arg Tyr Leu Lys Gly Trp Leu Glu Asp Trp Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  19
<211>  244
<212>  PRT
<213>  Artificial Sequence

<220>
<223>  Retroaldolase V12Y F112I RA9558F shortened

<400>  19

Pro Arg Tyr Leu Lys Gly Trp Leu Glu Asp Tyr Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  20
<211>  244
<212>  PRT
<213>  Artificial Sequence

<220>
<223>  Retroaldolase V12P F112I RA9558F shortened

<400>  20

Pro Arg Tyr Leu Lys Gly Trp Leu Glu Asp Pro Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  21
<211>  244
<212>  PRT
<213>  Artificial Sequence

<220>
<223>  Retroaldolase V12A F112V L210A RA9558F shortened

<400>  21

Pro Arg Tyr Leu Lys Gly Trp Leu Glu Asp Ala Val Gln Leu Ser Leu

1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Val Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Ala Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  22
<211>  244
<212>  PRT
<213>  Artificial Sequence

<220>
<223>  Retroaldolase F112I L210A RA9558F shortened

<400>  22

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Ile Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Ala Leu Asp Phe Phe Glu Pro Asn Glu Ile Glu Glu Leu Arg Lys Leu

210                 215                 220

Gly Val Asn Ala Phe Met Ile Ser Ser Ser Leu Met Arg Asn Pro Glu
225                 230                 235                 240

Lys Ile Lys Glu

<210>  23
<211>  244
<212>  PRT
<213>  Artificial Sequence

<220>
<223>  Retroaldolase I133F RA9558F shortened

<400>  23

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Phe Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  24
<211>  244
<212>  PRT
<213>  Artificial Sequence

<220>
<223>  Retroaldolase F184V RA9558F shortened

<400>  24

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Val Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  25
<211>  244
<212>  PRT
<213>  Artificial Sequence

<220>
<223>  Retroaldolase F184Y RA9558F shortened

<400>  25

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Tyr Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  26
<211>  244
<212>  PRT
<213>  Artificial Sequence

<220>
<223>  Retroaldolase L210I RA9558F shortened

<400>  26

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile

35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Ile Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  27
<211>  244
<212>  PRT
<213>  Artificial Sequence

<220>
<223>  Retroaldolase L210A RA9558F shortened

<400>  27

Pro Arg Tyr Leu Lys Gly Trp Leu Glu Asp Val Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Phe Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Ala Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  28
<211>  244
<212>  PRT
<213>  Artificial Sequence

<220>
<223>  Retroaldolase V12I F112L RA9558F shortened

<400>  28

Pro Arg Tyr Leu Lys Gly Trp Leu Glu Asp Ile Val Gln Leu Ser Leu
1               5                   10                  15

Arg Arg Pro Ser Val His Ala Ser Arg Gln Arg Pro Ile Ile Ser Leu
            20                  25                  30

Asn Glu Arg Ile Leu Glu Phe Asn Lys Arg Asn Ile Thr Ala Ile Ile
        35                  40                  45

Ala Tyr Tyr Leu Arg Lys Ser Pro Ser Gly Leu Asp Val Glu Arg Asp
    50                  55                  60

Pro Ile Glu Tyr Ala Lys Tyr Met Glu Pro Tyr Ala Val Gly Leu Ser
65                  70                  75                  80

Ile Lys Thr Glu Glu Lys Tyr Phe Asp Gly Ser Tyr Glu Met Leu Arg
                85                  90                  95

Lys Ile Ala Ser Ser Val Ser Ile Pro Ile Leu Met Asn Asp Leu Ile
            100                 105                 110

Val Lys Glu Ser Gln Ile Asp Asp Ala Tyr Asn Leu Gly Ala Asp Thr
        115                 120                 125

Val Leu Leu Ile Val Glu Ile Leu Thr Glu Arg Glu Leu Glu Ser Leu
    130                 135                 140

Leu Glu Tyr Ala Arg Gly Tyr Gly Met Glu Pro Leu Ile Leu Ile Asn
145                 150                 155                 160

Asp Glu Asn Asp Leu Asp Ile Ala Leu Arg Ile Gly Ala Arg Phe Ile
                165                 170                 175

Thr Ile Tyr Ser Met Asn Phe Glu Thr Gly Glu Ile Asn Lys Glu Asn
            180                 185                 190

Gln Arg Lys Leu Ile Ser Met Ile Pro Ser Asn Val Val Lys Val Pro
        195                 200                 205

Leu Leu Asp Phe Phe Glu Pro Asn Glu Ile Glu Glu Leu Arg Lys Leu
    210                 215                 220

Gly Val Asn Ala Phe Met Ile Ser Ser Ser Leu Met Arg Asn Pro Glu
225                 230                 235                 240

Lys Ile Lys Glu

<210>  29
<211>  13
<212>  PRT
<213>  Artificial Sequence

<220>
<223>  affinity tag/linker

<400>  29

Leu Ile Glu Gly Ser Leu Glu His His His His His His
1               5                   10

<210>  30
<211>  258
<212>  PRT
<213>  Artificial Sequence

<220>
<223>  Retroaldolase F112I RA9558F full length

<400>  30

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile

            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210> 31
<211> 258
<212> PRT
<213> Artificial Sequence

<220>
<223> Retroaldolase F112L RA9558F full length

<400> 31

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Leu
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  32
<211>  258
<212>  PRT
<213>  Artificial Sequence

<220>
<223>  Retroaldolase F112V RA9558F full length

<400>  32

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Val
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  33
<211>  258
<212>  PRT
<213>  Artificial Sequence

<220>
<223>  Retroaldolase F184V RA9558F full length

<400>  33

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Val Glu Thr Gly Glu Ile Asn Lys Glu
                180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  34
<211>  258
<212>  PRT
<213>  Artificial Sequence

<220>
<223>  Retroaldolase I133F RA9558F full length

<400>  34

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Phe Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  35
<211>  258
<212>  PRT
<213>  Artificial Sequence

<220>
<223>  Retroaldolase V12G F112I RA9558F full length

<400>  35

Met Pro Arg Tyr Leu Lys Gly Trp Leu Glu Asp Gly Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg

50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  36
<211>  258
<212>  PRT
<213>  Artificial Sequence

<220>
<223>  Retroaldolase V12F F112I RA9558F full length

<400>  36

Met Pro Arg Tyr Leu Lys Gly Trp Leu Glu Asp Phe Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His

245                 250                 255

His His

<210>  37
<211>  258
<212>  PRT
<213>  Artificial Sequence

<220>
<223>  Retroaldolase V12I F112L RA9558F full length

<400>  37

Met Pro Arg Tyr Leu Lys Gly Trp Leu Glu Asp Ile Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Leu
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  38
<211>  258
<212>  PRT
<213>  Artificial Sequence

<220>
<223>  Retroaldolase V12A F112V RA9558F full length

<400>  38

Met Pro Arg Tyr Leu Lys Gly Trp Leu Glu Asp Ala Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Val
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  39
<211>  258
<212>  PRT
<213>  Artificial Sequence

<220>
<223>  Retroaldolase V12G RA9558F full length

<400>  39

Met Pro Arg Tyr Leu Lys Gly Trp Leu Glu Asp Gly Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  40
<211>  258
<212>  PRT
<213>  Artificial Sequence

<220>
<223>  Retroaldolase V12A RA9558F full length

<400>  40

Met Pro Arg Tyr Leu Lys Gly Trp Leu Glu Asp Ala Val Gln Leu Ser

1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  41
<211>  258
<212>  PRT
<213>  Artificial Sequence

<220>
<223>  Retroaldolase V12F RA9558F full length

<400>  41

Met Pro Arg Tyr Leu Lys Gly Trp Leu Glu Asp Phe Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val

195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  42
<211>  258
<212>  PRT
<213>  Artificial Sequence

<220>
<223>  Retroaldolase V12F F112L RA9558F full length

<400>  42

Met Pro Arg Tyr Leu Lys Gly Trp Leu Glu Asp Phe Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Leu
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  43
<211>  258
<212>  PRT
<213>  Artificial Sequence

<220>
<223>  Retroaldolase V12A F112L RA9558F full length

<400>  43

Met Pro Arg Tyr Leu Lys Gly Trp Leu Glu Asp Ala Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Leu
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  44
<211>  258
<212>  PRT
<213>  Artificial Sequence

<220>
<223>  Retroaldolase V12L F112V RA9558F full length

<400>  44

Met Pro Arg Tyr Leu Lys Gly Trp Leu Glu Asp Leu Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Val
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210> 45
<211> 258
<212> PRT
<213> Artificial Sequence

<220>
<223> Retroaldolase V12W F112V RA9558F full length

<400> 45

Met Pro Arg Tyr Leu Lys Gly Trp Leu Glu Asp Trp Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Val
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  46
<211>  258
<212>  PRT
<213>  Artificial Sequence

<220>
<223>  Retroaldolase V12W F112I RA9558F full length

<400>  46

Met Pro Arg Tyr Leu Lys Gly Trp Leu Glu Asp Trp Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile

145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  47
<211>  258
<212>  PRT
<213>  Artificial Sequence

<220>
<223>  Retroaldolase V12Y F112I RA9558F full length

<400>  47

Met Pro Arg Tyr Leu Lys Gly Trp Leu Glu Asp Tyr Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  48
<211>  258
<212>  PRT
<213>  Artificial Sequence

<220>
<223>  Retroaldolase V12P F112I RA9558F full length

<400>  48

Met Pro Arg Tyr Leu Lys Gly Trp Leu Glu Asp Pro Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  49
<211>  258

<212>  PRT
<213>  Artificial Sequence

<220>
<223>  Retroaldolase V12A F112V L210A RA9558F full length

<400>  49

Met Pro Arg Tyr Leu Lys Gly Trp Leu Glu Asp Ala Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Val
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Ala Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  50
<211>  258
<212>  PRT
<213>  Artificial Sequence

<220>
<223>  Retroaldolase F112I L210A RA9558F full length

<400>  50

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Ile
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Ala Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  51
<211>  258
<212>  PRT
<213>  Artificial Sequence

<220>
<223>  Retroaldolase F184Y RA9558F full length

<400>  51

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe

100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Tyr Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Leu Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  52
<211>  258
<212>  PRT
<213>  Artificial Sequence

<220>
<223>  Retroaldolase L210I RA9558F full length

<400>  52

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Ile Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

<210>  53
<211>  258
<212>  PRT
<213>  Artificial Sequence

<220>
<223>  Retroaldolase L210A RA9558F full length

<400>  53

Met Pro Arg Tyr Leu Lys Gly Trp Leu Glu Asp Val Val Gln Leu Ser
1               5                   10                  15

Leu Arg Arg Pro Ser Val His Ala Ser Arg Gln Arg Pro Ile Ile Ser
            20                  25                  30

Leu Asn Glu Arg Ile Leu Glu Phe Asn Lys Arg Asn Ile Thr Ala Ile
        35                  40                  45

Ile Ala Tyr Tyr Leu Arg Lys Ser Pro Ser Gly Leu Asp Val Glu Arg
    50                  55                  60

Asp Pro Ile Glu Tyr Ala Lys Tyr Met Glu Pro Tyr Ala Val Gly Leu
65                  70                  75                  80

Ser Ile Lys Thr Glu Glu Lys Tyr Phe Asp Gly Ser Tyr Glu Met Leu
                85                  90                  95

Arg Lys Ile Ala Ser Ser Val Ser Ile Pro Ile Leu Met Asn Asp Phe
            100                 105                 110

Ile Val Lys Glu Ser Gln Ile Asp Asp Ala Tyr Asn Leu Gly Ala Asp
        115                 120                 125

Thr Val Leu Leu Ile Val Glu Ile Leu Thr Glu Arg Glu Leu Glu Ser
    130                 135                 140

Leu Leu Glu Tyr Ala Arg Gly Tyr Gly Met Glu Pro Leu Ile Leu Ile
145                 150                 155                 160

Asn Asp Glu Asn Asp Leu Asp Ile Ala Leu Arg Ile Gly Ala Arg Phe
                165                 170                 175

Ile Thr Ile Tyr Ser Met Asn Phe Glu Thr Gly Glu Ile Asn Lys Glu
            180                 185                 190

Asn Gln Arg Lys Leu Ile Ser Met Ile Pro Ser Asn Val Val Lys Val
        195                 200                 205

Pro Ala Leu Asp Phe Phe Glu Pro Asn Glu Ile Glu Glu Leu Arg Lys
    210                 215                 220

Leu Gly Val Asn Ala Phe Met Ile Ser Ser Ser Leu Met Arg Asn Pro
225                 230                 235                 240

Glu Lys Ile Lys Glu Leu Ile Glu Gly Ser Leu Glu His His His His
                245                 250                 255

His His

## Description

The present invention is directed to methods for catalyzing a chemical reaction by retroaldolases, corresponding uses of retroaldolases and to novel retroaldolases. The methods and retroaldolases have utility in (i) preparing tertiary alcohols, in (ii) chiral resolution of tertiary alcohols by retroaldol cleavage, and in (iii) deuteration or tritiation of carbonyl compounds.

Tertiary alcohols, in particular chiral tertiary alcohols, are common motifs in natural compounds and pharmaceuticals, as well as very valuable synthetic intermediates for the preparation of tertiary amines and enantiopure quaternary carbons. The stereocontrolled synthesis of chiral tertiary alcohols is typically based on the controlled attack of a carbanion on a prochiral ketone, for which chemical methods based on organometallic reagents have difficulties achieving a broad substrate scope and high enantio- and diastereoselectivity. In contrast, the utilization of suitable enzyme catalysts could provide very high stereoselectivity, low and economic costs, and more environmentally benign conditions.

Biocatalytic procedures based on hydrolases have been developed for the resolution of racemic tertiary alcohols, yet synthetic asymmetric reactions would be more cost- and waste-efficient. For example, US 5912355 discloses a method for the resolution of racemic tertiary alcohols using hydrolases. However, this process is far less efficient than the asymmetric synthesis of the desired compounds. In research environments, chiral tertiary alcohols have been synthesized using diverse classes of enzymes, including hydrolases, oxygenases, terpene synthases, cyclases and distinct types of lyases (see, e.g., ChemBioEng Rev 2014, 1, No. 1, 14-26).

Most of these enzymes, however, display poor tolerance towards substrates different than those they use in nature. Some hydroxynitrile lyases-which catalyze the reversible addition of cyanide to carbonyl groups - present a broader substrate scope, but their attractiveness is hindered by high background reaction levels, the utilization of large concentrations of toxic cyanide salts, and limited functional diversity. Enzymes are broadly used in the industrial synthesis of secondary alcohols, but, as a result of the above and other drawbacks, only a very limited number of enzymes have been reported for the generation of tertiary alcohols (see, e.g., Curr Opin Chem Biol. 2013 Apr; 17(2):221-8).

For example, EP 1719758 discloses a method to catalyze the aldol addition of pyruvic acid and 3-(1H-indol-3-yl)-2-oxopropanoic acid to yield a chiral tertiary alcohol with a natural aldolase. However, this enzyme is highly substrate-specific and possibly not suitable for the generation of tertiary alcohols with other substrates that the enzyme does not naturally accept.

Müller et al. (Adv. Synth. Catal. 2012, 354, 3161-3174) report aldolase-catalyzed asymmetric C-C bond formation reactions. With regard to the formation of tertiary alcohols, Müller et al. conclude that aldolases share the drawback that (a) the equilibrium of the reaction they catalyze is far on the side of the cleavage products, rather than on the side of the tertiary alcohol, and (b), many aldolases require cofactors, e.g. CoA, which is not practical for application on a preparative scale.

The present inventors have previously reported (Nature Chemistry 2017, 9, 50-56) that two computationally designed and artificially modified retroaldolases (RA95.5-8F and RA95.5-8, SEQ ID NOS: 1 and 2) catalyze the stereoselective reaction of an aromatic or aliphatic aldehyde with acetone to generate secondary alcohols. The two retroaldolases also catalyze the stereoselective cleavage of racemic beta-hydroxycarbonyl compounds into acetone and an aromatic aldehyde. Modifications that lead to the above retroaldolases included random mutagenesis of the entire gene, cassette mutagenesis of the desired sites and DNA shuffling of verified mutants. Natural aldolases that use aldehydes as electrophiles have not been reported to tolerate ketones as electrophiles.

In addition to stereoselective aldol reactions, deuterations or tritiations are important reactions, e.g., in research for protein structure analysis and the preparation of metabolic probes. Known deuteration procedures are based on the catalytic aldolase antibody 38C2 which catalyzes deuteration of a range of carbonylic compounds with high reaction kinetics (Chem. Eur. J. 2002, 8, 229-239). The selectivity of the catalyst, however, is not always satisfactory. The deuteration of cyclohexanone, for example, is fast and regioselective, but the catalyst eventually overruns the desired scope of the reaction and exchanges the four hydrogens of C-2 and C-6.

It is the objective of the present invention to provide means for catalyzing the generation of tertiary alcohols and preferably for catalyzing deuteration or tritiation reactions and/or chiral resolution reactions.

In a first aspect, the present invention is directed to a method for catalyzing a chemical reaction selected from the group consisting of:
(i) preparing tertiary alcohols, preferably chiral tertiary alcohols, by an aldol reaction;
(ii) chiral resolution of tertiary alcohols by retroaldol cleavage; and
(iii) deuteration or tritiation of carbonyl compounds, preferably at the α-position of the carbonyl group of carbonyl compounds, more preferably regio- and/or stereoselective deuteration or tritiation of carbonyl compounds, most preferably with the *proviso* that the carbonyl compounds are not acetone,
comprising the steps of:
(a) providing a retroaldolase, preferably in solution, as a lyophilized powder, or immobilized on a solid phase, which retroaldolase is selected from the group consisting of:
   (aa) a retroaldolase comprising, preferably having, an amino acid sequence selected from the group consisting of SEQ ID NOs: 3 to 28;
   (ab) a retroaldolase comprising, preferably having, an amino acid sequence having an amino acid sequence identity of at least 70% or 80%, preferably at least 90 or 95%, most preferably 95% or 98% with an amino acid sequence selected from the group consisting of SEQ ID NOs: 3 to 28;
   (ac) a retroaldolase comprising a functional derivative and/or functional fragment of (aa) and/or (ab); and
   (ad) a retroaldolase according to any of (aa) to (ac), wherein in SEQ ID NOs: 3 to 28, position 50 is tyrosine, position 82 is lysine and position 109 is asparagine and/or position 179 is preferably tyrosine, more preferably phenylalanine, preferably the retroaldolase comprising, more preferably having, SEQ ID NO: 30, or the retroaldolase comprising, preferably having, SEQ ID NO: 34, a functional derivative and/or functional fragment of these, for catalyzing the chemical reaction (i), (ii) or (iii),
(b) providing at least one substrate for the chemical reaction (i), (ii) or (iii) selected from the group consisting of
   (ba) (baa) an aldehyde-comprising substrate and a ketone-comprising substrate, preferably a nucleophilic aldehyde-comprising substrate and an electrophilic ketone-comprising substrate, or (bab) two ketone-comprising substrates, which substrates react in the aldol reaction (i) to form a tertiary alcohol;
   (bb) tertiary alcohols for chiral resolution by retroaldol cleavage (ii); and
   (bc) carbonyl compounds, preferably with the *proviso* that the carbonyl compounds are not acetone, for deuteration or tritiation (iii);
(c) contacting the retroaldolase of (a) with the substrate of (b) under conditions that allow enzymatic activity of the retroaldolase and the chemical reaction to proceed, and
(d) optionally purifying the product of the chemical reaction.

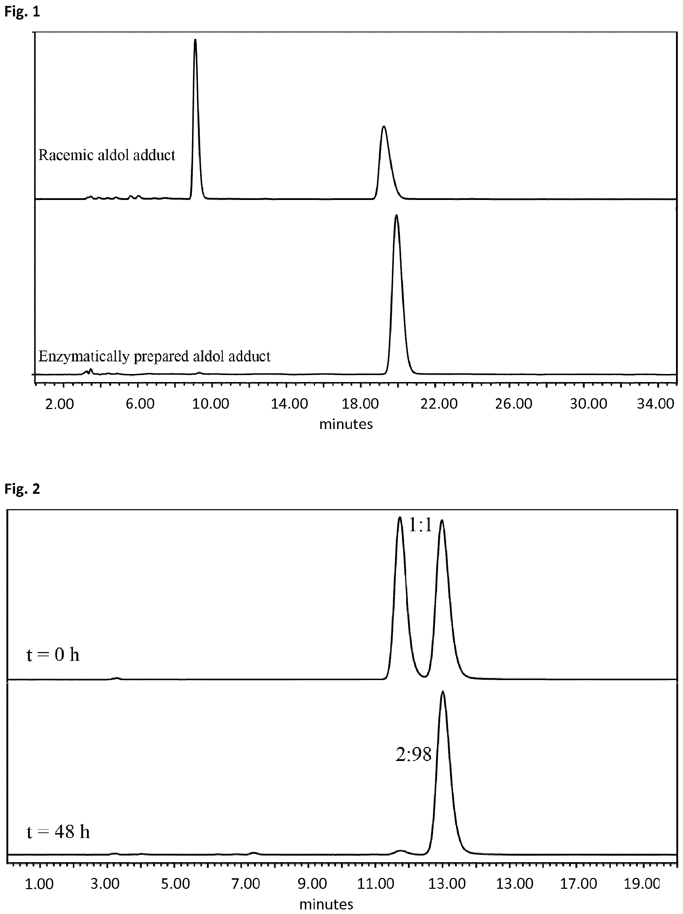

It was surprisingly found that the retroaldolase RA95.5-8F (SEQ ID NO: 1), preferably its core sequence having SEQ ID NO: 3, has utility in catalyzing the generation of tertiary alcohols, in particular chiral tertiary alcohols, from two ketone-comprising substrates or from an aldehyde-comprising substrate and a ketone-comprising substrate, preferably a nucleophilic aldehyde-comprising substrate and an electrophilic ketone-comprising substrate (see Example 1 and Fig. 1 below). Furthermore, it was found that retroaldolases, preferably modified retroaldolases, which comprise an amino acid sequence having at least 70%, preferably at least 90 or 95%, more preferably 95% or 98% sequence identity with the sequence of SEQ ID NO: 3 or SEQ ID NO: 4 are improved catalysts for the generation of tertiary alcohols, in particular chiral tertiary alcohols (see Example 2 below). Neither of the two retroaldolases RA95.5-8F (SEQ ID NO: 1) or RA95.5-8 (SEQ ID NO: 2), or their core sequences (SEQ ID NOs: 3, 4), have been reported to catalyze the formation of chiral tertiary alcohols. Also, no other retroaldolase, in particular no other artificial retroaldolase, has been reported to catalyze the formation of tertiary alcohols from two ketone-comprising substrates or a nucleophilic aldehyde and an electrophilic ketone.

The retroaldolases for use in step (a) of the method of the present invention catalyze the generation of chiral tertiary alcohols under high stereoselectivity, preferably enantioselectivity, provide high turnover numbers with little background reactions and can be used in mild reaction conditions, such as aqueous media and mild to low temperatures. Also, the retroaldolases for use in the present invention are inexpensive and environmentally benign to use. Furthermore, the retroaldolases described herein retain catalytic activity in some organic solvents, and preferably in emulsion systems. Furthermore, the retroaldolases described herein are, preferably, not sensitive towards oxygen. The aldolases are preferably active in aqueous solutions, as a suspension of lyophilized powder, and/or immobilized on solid phases.

A further advantage of the retroaldolases for use in the present invention is that they do not require cofactors. Furthermore, the retroaldolases for use in the present invention accept a wide variety of ketone-comprising substrates for the aldol reaction. It is preferred that the electrophile substrate of the retroaldolase for use in the present invention comprise at least one electron-withdrawing group that activates the electrophile, such as, for example, esters, amides, NO₂-substituted aromatic rings, thioesters, 1,3-oxazolines or halides. Preferably, the electron-withdrawing group is in α-position of the ketone. The retroaldolases for use in the present invention generally distinguish between the electrophile and the nucleophile which leads to the generation of only one desired aldol product under high selectivity and few to no cross-reaction products between electrophile/electrophile or nucleophile/nucleophile.

The term "retroaldolase" as used herein is meant to describe an enzyme, i.e. a (poly)-peptide, having catalytic activity in at least catalyzing the preparation of tertiary alcohols, preferably chiral tertiary alcohols, by an aldol reaction, which is a reaction from two carbonyl-comprising molecules, wherein the tertiary alcohol is formed from one of the carbonyl moieties of the carbonyl-comprising substrates. The carbonyl-comprising compounds reacting in the aldol reaction can either be a ketone-comprising substrate and an aldehyde-comprising substrate, or two ketone-comprising substrates, as long as they react to a tertiary alcohol. In the case of a ketone-comprising substrate and an aldehyde-comprising substrate reacting in the aldol reaction, it is preferred that the aldehyde-comprising substrate is nucleophilic and the ketone-comprising substrate is electrophilic. The terms "nucleophile/nucleophilic" and "electrophile/- electrophilic" as used herein, are meant to describe the electronic properties of the substrates in relation to one another. In other words, the nucleophile is the substrate that donates an electron pair to the electrophile to form the aldol bond.

Also, the term "retroaldolase" is meant to encompass the term "aldolase". The retroaldolases described herein preferably also have catalytic activity for catalyzing a reaction selected from the group consisting of (i) chiral resolution of tertiary alcohols by retroaldol cleavage; and (ii) deuteration or tritiation of a carbonyl compound, preferably at the α-position of the carbonyl group of the carbonyl compound, more preferably regio- and/or stereoselective deuteration or tritiation of a carbonyl compound, most preferably with the *proviso* that the carbonyl compound is not acetone. Both of these reactions are believed to be based on the retroaldolases' ability to selectively activate the ketone-comprising reactants to catalyze the reactions. The term "retro-aldol cleavage" as used herein is meant to refer to the reverse reaction of an aldol reaction.

The term "catalyze" as used herein means that the retroaldolase for use in the present invention increases the rate of the reaction towards the desired product, i.e. towards the aldol product, the resolution product(s) or the deuterated or tritiated product, to a greater extent compared to the rate of the reaction in the absence of the retroaldolase, and preferably also with a higher stereoselectivity compared to the reaction in the absence of the retroaldolase.

The term "chiral resolution of tertiary alcohols" as used herein is meant to encompass any enzymatic cleavage of tertiary alcohols, preferably any enzymatic cleavage of one stereoisomer to a larger extent than the other stereoisomer, preferably selectively only one stereoisomer of a tertiary alcohol, as exemplified in representative Example 3 and Fig. 2 below. The tertiary alcohol can be any organic compound that comprises a tertiary hydroxyl group which can be cleaved by a retroaldolase for use in the present invention. Of course, the tertiary alcohols that are used for the chiral resolution are not enantiopure before the resolution reactions, which means that the tertiary alcohols can be racemates or any mixture, e.g. an enantioenriched mixture, of stereoisomers, preferably enantio- or diastereomeric mixtures of the tertiary alcohols.

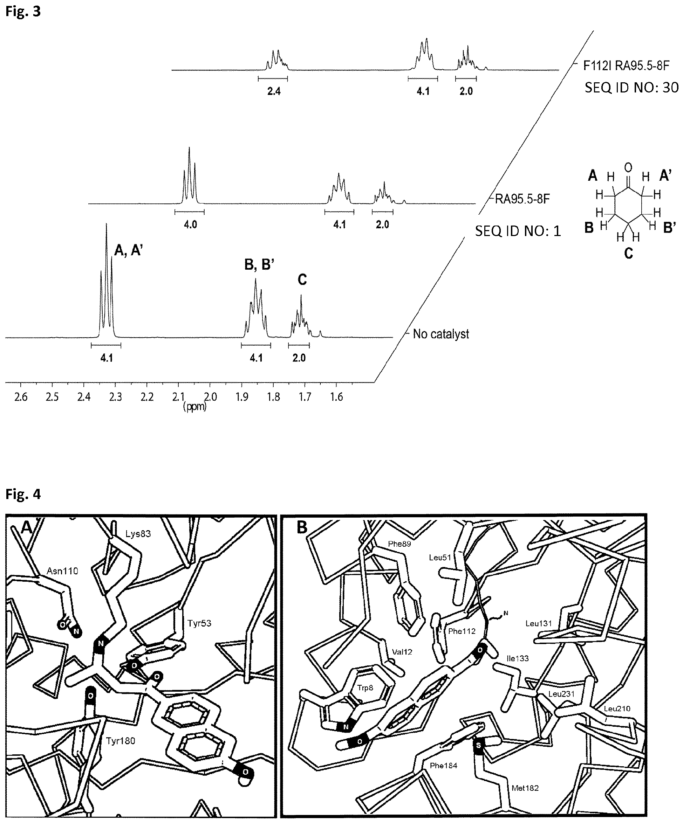

The term "deuteration or tritiation of carbonyl compounds" as used herein refers to the installation of a deuterium or tritium, preferably in alpha-position, of the carbonyl functionality of a carbonyl-comprising organic compound, as exemplified in representative Example 3 and Fig. 3 below. Without wishing to be bound by theory, the retroaldolases for use in the present invention are believed to stabilize enamine intermediates of carbonyl compounds and are thus able to introduce hydrogen isotopes at the alpha-position(s) of the carbonyl group, preferably in a regio- and stereoselective manner. Deuteration reactions can also be used for the kinetic characterization of (retro)aldolase catalysts. Of course, the retroaldolases for use in the present invention can also exchange deuterium or tritium with ¹H hydrogen.

The term "(poly)peptide" as used herein is meant to comprise peptides, polypeptides, oligopeptides and proteins that comprise two or more amino acids linked covalently through peptide bonds. The term does not refer to a specific length of the product. (Poly)peptides include post-translational modifications of the (poly)peptides, for example, glycosylations, acetylations, phosphorylations, cleavages and the like. The term preferably also encompasses (poly)peptide analogs, (poly)peptides comprising non-natural amino acids, peptidomimetics, β-amino acids, etc.

The percentage identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

The term "functional derivative" of a (poly)peptide described in the present invention is meant to include any (poly)peptide or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative still has at least some retroaldolase activity to a measurable extent, e.g. of at least about 1 to 10 %, preferably at least about 20 to 50 % aldolase activity of the unmodified (poly)peptide, e.g. a retroaldolase comprising SEQ ID NO: 1, for use in the invention.

In this context a "functional fragment" as used herein is one that forms part of a (poly)peptide or derivative of the invention and still has at least some retroaldolase activity to a measurable extent, e.g. of at least about 1 to 10 %, preferably at least about 20 to 50 % retroaldolase activity of the unmodified (poly)peptide, e.g. a retroaldolase comprising SEQ ID NO: 1, for use the invention.

The term "(poly)peptide" as used herein also encompasses an isolated and purified (poly)-peptide. The term "isolated and purified (poly)peptide", as used herein, refers to a (poly)peptide or a peptide fragment which either has no naturally-occurring counterpart (e.g., a peptidemimetic), or has been separated or purified from components which naturally accompany it. Preferably, a (poly)peptide is considered "isolated and purified" when it makes up for at least 60 % (w/w) of a dry preparation, thus being free from most naturally-occurring (poly)peptides and/or organic molecules with which it is naturally associated. Preferably, a (poly)peptide of the inventtion makes up for at least 80%, more preferably at 90%, and most preferably at least 99% (w/w) of a dry preparation. More preferred are (poly)peptides according to the invention that make up for at least at least 80%, more preferably at least 90%, and most preferably at least 99% (w/w) of a dry (poly)peptide preparation. Chemically synthesized (poly)peptides are by nature "isolated and purified" within the above context.

An isolated (poly)peptide as described herein may be obtained as discussed below, e.g. in Examples 5-9, and /or, for example, by expression of a recombinant nucleic acid encoding the (poly)peptide in a host, preferably a heterologous host, more preferably *E. coli*; or by chemical synthesis. A (poly)peptide that is produced in a cellular system being different from the source from which it naturally originates is "isolated and purified", because it is separated from components which naturally accompany it. The extent of isolation and/or purity can be measured by any appropriate method, e.g., column chromatography, polyacrylamide gel electrophoresis, HPLC analysis, NMR spectroscopy, gas liquid chromatography, or mass spectrometry.

The retroaldolases for use in the present invention can be specifically modified and tailored to the desired substrates, preferably to the nucleophile, more preferably to substrate size and polarity, according to standard protocols for enzyme evolution, e.g. by a microfluidics-based assay, which is commonly used in the field for the evolution of enzymes (see e.g. Nat. Chem. 2017, 9, 50-56, further below and Examples 5-9).

Without wishing to be bound by theory, the retroaldolases for use in the present invention share a catalytic core comprised of the amino acids at positions 50 (tyrosine), 82 (lysine) and 109 (asparagine) of SEQ ID NOs: 3 to 28. Also, position 179 of SEQ ID NOs: 3 to 28 is involved in the catalytic reaction and preferably is tyrosine or phenylalanine, yet position 179 is believed not to be essential. The catalytic core of RA95-8F (SEQ ID NO: 1) is shown in Figs. 4A-B. Furthermore, the N-terminal methionine in position 1 of the SEQ ID NOs: 1 and 2 is - as commonly known in the art-typically cleaved post-translationally and is therefore believed not to be essential; residues 246-258 of SEQ ID NOs: 1 and 2 are believed not to be functional and correspond to an affinity tag and a linker.

It is preferred that the present invention also includes retroaldolases as such (compounds, compositions), for use in the methods and for the use as described herein, which retroaldolases comprise amino acid sequences according to SEQ ID NOs: 3 to 28, wherein before position 1 (N-terminal) of SEQ ID NOs: 3 to 28, a methionine is present, and/or wherein a residue sequence according to SEQ ID NO: 29 is present at the C-terminus of SEQ ID NOs: 3 to 28 (i.e. in positions 246-258 if a methionine is present or positions 245-257 if no methionine is present). For example, a retroaldolase as described herein comprising SEQ ID NO: 3, wherein a methionine is present before position 1 of SEQ ID NO: 3, and wherein a residue sequence having SEQ ID NO: 29 is present at the C-terminus of SEQ ID NO: 3 corresponds to a retroaldolase comprising SEQ ID NO: 1. Retroaldolases comprising SEQ ID NOs: 3 to 28 that feature a methionine before position 1 and the residue having SEQ ID NO: 29 at the C-terminus are expressly included as preferred embodiments of all aspects of the present invention and their sequences are disclosed as SEQ ID NOs: 1, 2, and 30 to 53. Of course, the amino acids at positions 50 (tyrosine), 82 (lysine), 109 (asparagine) and 179 of SEQ ID NOs: 3 to 28 correspond to the amino acids at positions 51 (tyrosine), 83 (lysine), 110 (asparagine) and 180 if a methionine is present, e.g. in SEQ ID NOs: 1, 2 and 30 to 53.

In step (b), the substrates of the reactions are as defined above (see definitions of "retroaldolase", "chiral resolution of tertiary alcohols" and "deuteration or tritiation of carbonyl compounds").

It is preferred that the nucleophile substrate of the aldol reaction is present in excess of the electrophile substrate, or, more preferably, the nucleophile substrate and the electrophile substrate are present at equimolar concentrations. Preferably, the nucleophile substrate and electrophile substrate are used at molar ratios of (nucleophile to electrophile) 99:1 to 1:99, preferably 80:20 to 20:80, more preferably 60:40 to 40:60, most preferably about 1:1. Suitable amounts of the retroaldolase for catalyzing the reaction according to the method of the inventtion are 0.1 to 50 molar equivalents to the electrophile, preferably 0.1 to 10 molar equivalents to the electrophile, more preferably 0.1 to 5 molar equivalents to the electrophile, most preferably 0.1 to 1 molar equivalents to the electrophile.

Suitable conditions in step (c) include all conditions that allow for the desired reaction to take place and for the retroaldolase to exert its catalytic activity. Such conditions are known in the art and include, e.g., conditions noted in Examples 1 and 2 below, and aqueous solutions containing buffering agents such as, e.g., HEPES, Tris-HCl, glycylglycine, borate salts, or phosphate salts, co-solvents such as dimethyl sulfoxide, tetrahydrofuran, methanol, ethanol, isopropanol, or acetonitrile, emulsion systems etc. Suitable temperatures include temperature ranges from 10 to 50 °C, preferably from 20 to 30 °C.

The optional purification in step (d) can be carried out with commonly known methods, such as extraction, column chromatography, including silica columns, reverse-phase columns and HPLC, crystallization, distillation, and/or by the methods described in the Examples below.

It was surprisingly found that preferably positions 11, 111, 132, 183 and 209 of SEQ ID NOs: 3 to 28, preferably of SEQ ID NO: 3, can be modified to tailor the retroaldolases for use in the present invention to different substrates (see Example 2 below). Of course, the amino acids at positions 11, 111, 132, 183 and 209 of SEQ ID NOs: 3 to 28 correspond to the amino acids at positions 12, 112, 133, 184 and 210 if a methionine is present, e.g. in SEQ ID NOs: 1, 2 and 30 to 53.

In a preferred embodiment, the method of the present invention further comprises step (f) of modifying the retroaldolase of (a), preferably in at least one of positions 11, 111, 132, 183 and 209 of SEQ ID NO: 3 to 28, preferably of SEQ ID NO: 3, wherein step (f) is performed after step (a) and before step (c).

Step (f) of modifying the retroaldolase and any reference herein to modifying the retroaldolase includes specifically modifying and tailoring the retroaldolase to the desired substrates, preferably to the nucleophile, more preferably to substrate size and polarity, according to standard protocols for enzyme evolution, e.g. by (A) generating a library of retroaldolases by random mutagenesis of the entire gene (e.g. error-prone PCR), cassette mutagenesis of the desired sites and DNA shuffling of verified mutants, (B) microfluidics or/and microtiter-plate based screening assay, and/or (C) crystallization and structure determination, all of which (A) to (C) are commonly used in the field for the evolution of enzymes, as also noted above (see e.g. Nat. Chem. 2017, 9, 50-56, in particular page 55 "Methods", further below and Examples 5-9). Also, modification in step (f) can be performed as discussed in the preferred embodiment above.

It is noted that any modification of the retroaldolase, including the modification in optional step (f) above or in the method of modifying a retroaldolase below, is meant to lead to (i) a retroaldolase comprising, preferably having, an amino acid sequence selected from the group consisting of SEQ ID NOs: 3 to 28, (ii) a retroaldolase comprising, preferably having, an amino acid sequence having an amino acid sequence identity of at least 70% or 80%, preferably at least 90 or 95%, most preferably 95% or 98% with SEQ ID NO: 3 to 28, (iii) a retroaldolase comprising a functional derivative and/or functional fragment of (i) and/or (ii), and/or (iv) to a retroaldolase according to any of (i) to (iii), wherein in SEQ ID NOs: 3 to 28, position 50 is tyrosine, position 82 is lysine and position 109 is asparagine and/or position 179 is preferably tyrosine, more preferably phenylalanine.

In a further aspect, the present invention is directed to a method for modifying a retroaldolase for catalyzing a chemical reaction selected from the group consisting of:
(i) preparing tertiary alcohols, preferably chiral tertiary alcohols, by an aldol reaction;
(ii) chiral resolution of tertiary alcohols by retroaldol cleavage; and
(iii) deuteration or tritiation of carbonyl compounds, preferably at the α-position of the carbonyl group of the carbonyl compounds, more preferably regio- and/or stereoselective deuteration or tritiation of carbonyl compounds, most preferably with the *proviso* that the carbonyl compounds are not acetone,
comprising the steps of:
(a) providing a retroaldolase selected from the group consisting of
   a. a retroaldolase comprising, preferably having, an amino acid sequence selected from the group consisting of SEQ ID NOs: 3 to 28;
   b. a retroaldolase comprising, preferably having, an amino acid sequence having an amino acid sequence identity of at least 70% or 80%, preferably at least 90 or 95%, most preferably 95% or 98% with an amino acid sequence selected from the group consisting of SEQ ID NOs: 3 to 28;
   c. a retroaldolase comprising a functional derivative and/or functional fragment of a. and/or b.; and
   d. a retroaldolase according to any of a. to c., wherein in SEQ ID NOs: 3 to 28, position 50 is tyrosine, position 82 is lysine and position 109 is asparagine and/or position 179 is preferably tyrosine, more preferably phenylalanine;
(b) modifying at least one amino acid position, preferably at least one of positions 11, 111, 132, 183 and 209 of SEQ ID NO: 3 to 28 in any one of the above retroaldolases a. to d.;
(c) providing at least one substrate of interest for at least one of the above reactions (i) to (iii), and
(d) contacting the at least one substrate of interest of (c) with at least one of the modified retroaldolases a. to d. under conditions that allow enzymatic activity of the retroaldolase and the reaction to proceed, and
(e) identifying at least one modified retroaldolase that catalyzes, preferably stereospecifically catalyzes at least one of the reactions (i) to (iii).

The steps (b), (c) and (d) of the above method are performed as defined for the method for catalyzing the chemical reaction.

Identifying the at least one modified retroaldolase in step (e) can be performed by commonly known characterization methods, e.g. as described in the Examples below. Suitable methods for carrying out the modifying method above include known techniques such as (A) microfluidics-based screening assay, and/or (B) crystallization and structure determination, all of which (A) and (B) are commonly used in the field for the evolution of enzymes, as also noted above (see e.g. Nat. Chem. 2017, 9, 50-56, in particular page 55 "Methods", further below and Examples 5-9).

In a preferred embodiment, the methods of the present invention are those, wherein in step (f) of the method for catalyzing a chemical reaction or in or step (b) of the method of modifying the retroaldolase, the retroaldolase is modified in one or more of the following positions of SEQ ID NO: 3 to 28, preferably in SEQ ID NO: 3 in position 11 by glycine, phenylalanine or alanine; in position 111 by isoleucine, leucine or valine; in position 132 by phenylalanine; in position 183 by valine or tyrosine; and/or in position 209 by isoleucine or alanine.

The above modifications have all been shown to lead to reactive and stereoselective retroaldolases for use in the present invention (see Example 2 below).

It is preferred that the methods of the present inventions are those, wherein the retroaldolase is selected from the group consisting of
(a) a retroaldolase comprising, preferably having, an amino acid sequence according to SEQ ID NOs: 5 to 28;
(b) a retroaldolase comprising, preferably having, an amino acid sequence having an amino acid sequence identity of at least 70% or 80%, preferably at least 90 or 95%, most preferably 95% or 98% with SEQ ID NOs: 5 to 28; and
(c) a retroaldolase comprising functional fragments and/or functional derivatives of any of (a) and/or (b).

In a further aspect, the present invention is directed to the use of a retroaldolase selected from the group consisting of
(a) a retroaldolase comprising, preferably having, an amino acid sequence selected from the group consisting of SEQ ID NOs: 3 to 28;
(b) a retroaldolase comprising, preferably having, an amino acid sequence having an amino acid sequence identity of at least 70% or 80%, preferably at least 90 or 95%, most preferably 95% or 98% with an amino acid sequence selected from the group consisting of SEQ ID NOs: 3 to 28; and
(c) a retroaldolase comprising a functional derivative and/or functional fragment of (a) and/or (b),
(d) a retroaldolase according to any of (a) to (c), preferably (a) to (b), wherein in SEQ ID NO:3 to 28, position 50 is tyrosine, position 82 is lysine and position 109 is asparagine and/or position 179 is preferably tyrosine, more preferably phenylalanine,
for catalyzing a chemical reaction selected from the group consisting of:
(i) preparing tertiary alcohols, preferably chiral tertiary alcohols, by an aldol reaction;
(ii) chiral resolution of tertiary alcohols by retroaldol cleavage; and
(iii) deuteration or tritiation of carbonyl compounds, preferably at the α-position of the carbonyl group of the carbonyl compounds, more preferably regio- and/or stereoselective deuteration or tritiation of carbonyl compounds, most preferably with the *proviso* that the carbonyl compounds are not acetone.

All definitions and explanations provided above in the context of the methods of the present invention also apply to the use of a retroaldolase as described herein.

In a preferred embodiment, the use of the retroaldolase according to the present inventtion is one, wherein the retroaldolase is modified in one or more of positions 11, 111, 132, 183 and 209 of SEQ ID NOs: 3 to 28, preferably of SEQ ID NO: 3, preferably is modified in position 11 by glycine, phenylalanine or alanine; in position 111 by isoleucine, leucine or valine; in position 132 by phenylalanine; in position 183 by valine or tyrosine; in position 209 by isoleucine or alanine; and/or combinations thereof.

In a further preferred embodiment, the use of the retroaldolase according the present invention is one, wherein the retroaldolase is selected from the group consisting of
(a) a retroaldolase comprising, preferably having, an amino acid sequence according to SEQ ID NOs: 5 to 28;
(b) a retroaldolase comprising, preferably having, an amino acid sequence having an amino acid sequence identity of at least 70% or 80%, preferably at least 90 or 95%., most preferably 95% or 98% with SEQ ID NOs: 5 to 28; and
(c) a retroaldolase comprising functional fragments and/or functional derivatives of any of (a) and/or (b).

In another preferred embodiment, the methods or the use according to the present invention are those, wherein the aldol reaction and/or the deuteration or tritiation reaction is a stereospecific reaction, preferably a diastereospecific and/or enantiospecific reaction.

The terms "stereospecific(ally)", "diastereospecific(ally)" and "enantiospecific(ally)", as used herein, mean that in the given reaction, one stereoisomer, diastereomer or enantiomer is generated in excess of the other, preferably one stereoisomer, diastereomer or enantiomer is generated in a molar amount of more than 50% compared to the other stereoisomer, diastereomer or enantiomer.

In a further preferred embodiment, the methods or the use according to the present invention are those, wherein the aldol reaction provides the tertiary alcohols diastereo-specifically and/or enantiospecifically, preferably provides the tertiary alcohols with a diastereomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and/or an enantiomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9.

The diastereomeric or enantiomeric ratio, as used herein, refers to the ratio the molar amount of one diastereoisomer or enantiomer in a mixture to the molar amount of the other diastereomer or enantiomer. A diastereomeric or entantiomeric ratio of, e.g., 1:2 means that one diastereomer or enantiomer is present at twice the molar amount of the other diastereomer or enantiomer.

In another preferred embodiment, the methods or the use according to the present invention are those, wherein the deuteration or tritiation reaction provides the deuterated or tritiated carbonyl compounds diastereospecifically and/or enantiospecifically, preferably provides the deuterated or tritiated carbonyl compounds with a diastereomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and/or an enantiomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9.

In a further preferred embodiment, the methods or the use according to the present invention are those, wherein the retroaldolase catalyzes the reaction 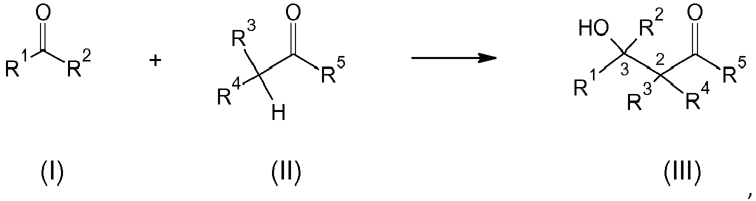
wherein at least one of R¹ or R² is an electron withdrawing residue, and
**R¹** and **R²** are independently selected from the group consisting of
   (i) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether, wherein the ether is bonded to formula (I) via its carbon atom;
   (ii) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
   (iii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl;
   (iv) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S; and
   (v) an electron withdrawing group, preferably selected from the group consisting of -COOR⁶, - CR⁷_{d}, -S(O)₂OH, -CONR¹R², wherein
      (aa) **R⁶** is selected from the group consisting of hydrogen, R¹, preferably substituted or non-substituted methyl, ethyl and propyl, most preferably methyl and ethyl;
      (bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
   wherein R¹ and/or R² are bonded directly to formula (I), via -O-, or via a -(CHₐ)_{b}- linker, wherein **a** is an integer from 0 to 2 and **b** is an integer from 1 to 10; preferably R¹ and/or R² are selected from the group consisting of -COOH, -COOMe, -COOEt, -CF₃, CHF₂, -CCl₃, and/or are bonded directly to Formula (I);
**R³** and **R⁴** are independently selected from the group consisting of
   (i) hydrogen, F, Cl, Br, R⁸, N(R⁸)e, OR⁸, S(R⁸), P(R⁸)_{f} and C(R⁸)_{d}, wherein **e** is 1 or 2, **f** is an integer from 1 to 4, **d** is an integer from 1 to 3, and **R⁸** is independently selected from the group consisting of
      (aa) hydrogen, F, Cl, Br, NO₂ and oxo;
      (bb) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether;
      (cc) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
      (dd) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl; and
      (ee) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S; and
   (ii) an electron withdrawing group, preferably selected from the group consisting of -COOR⁶, - CR⁷_{d}, -S(O)₂OH, -CONR¹R², wherein
      (aa) **R⁶** is selected from the group consisting of hydrogen, R¹, preferably substituted or non-substituted methyl, ethyl and propyl, most preferably methyl and ethyl;
      (bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein the electron withdrawing group is bonded directly to formula (II), vie -O-, or via a - (CHₐ)_{b}- linker, wherein **a** is an integer from 0 to 2 and **b** is an integer from 1 to 10; preferably the electron withdrawing group is selected from -COOH, -COOMe, -COOEt, -CF₃, CHF₂, -CCl₃, and/or is bonded directly to Formula (II);
**R⁵** is hydrogen or C(R⁸)_{d}, wherein **d** is an integer from 1 to 3, and **R⁸** is as defined above;
wherein the tertiary alcohol (III) is preferably a chiral tertiary alcohol and the stereogenic carbon atoms (2) and (3) of tertiary alcohol (III) are (*R,R*)*-,* (*S,R*)*-,* (*R,S*)- or (*S,S*)-configured, preferably with a diastereomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and/or an enantiomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9.

Preferred substrates for the methods or the use according to the present invention, wherein the retroaldolase catalyzes the chiral resolution of tertiary alcohols by retroaldol cleavage, are substrates as defined in Formula (III) above. It is further preferred that residues R¹ and/or R² of these substrates according to Formula (III) are selected from groups (i) to (iv), i.e. are not electron withdrawing groups.

Preferred substrates for the methods or the use according to the present invention, wherein the retroaldolase catalyzes the deuteration or tritiation of carbonyl compounds, are substrates as defined in Formula (II), wherein R³ and/or R⁴ are not hydrogen.

For compounds having asymmetric centers, it is understood that, unless otherwise specified, all of the optical isomers and mixtures thereof are encompassed. Each stereogenic carbon may be in the *(R)*- or *(S)*- configuration or a combination of configurations if not indicated differently. Also, compounds with two or more asymmetric elements can be present as mixtures of diastereomers. Furthermore, the compounds of the present invention preferably have a diastereomeric purity of at least 50% (1:1 mixture of diastereomers), preferably at least 60%, 70%, 80%, 85%, more preferably at least 90%, 95%, 96%, 97%, most preferably at least 98%, 99% or 100%. In addition, compounds with carbon-carbon double bonds may occur in *Z*- and *E*- forms, with all isomeric forms of the compounds being included in the present invention unless otherwise specified. Where a compound exists in various tautomeric forms, a recited compound is not limited to any one specific tautomer, but rather is intended to encompass all tautomeric forms.

Recited compounds are further intended to encompass compounds in which one or more atoms are replaced with an isotope, *i.e*., an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), may have an enantiomeric excess of at least 50%. For example, such compounds may have an enantiomeric excess of at least 60%, 70%, 80%, 85%, preferably at least 90%, 95%, or 98%. Some embodiments of the compounds have an enantiomeric excess of at least 99%. It will be apparent that single enantiomers (optically active forms), e.g. used as starting materials, can be obtained by asymmetric synthesis, synthesis from optically pure precursors, biosynthesis, or by resolution of the racemates, e.g. enzymatic resolution as described herein, resolution by conventional methods such as crystallization in the presence of a resolving agent, or chromatography, using, for example, a chiral HPLC column.

As used herein, a "substituent" or "residue" or "**R**", refers to a molecular moiety that is covalently bound to an atom within a molecule of interest. For example, a "substituent", "**R**" or "residue" may be a moiety such as a halogen, alkyl group, haloalkyl group or any other substituent described herein that is covalently bonded to an atom, preferably a carbon or nitrogen atom, that that forms part of a molecule of interest. The term "substituted" as used herein, means that any one or more hydrogens on the designated atom is replaced with a selection from the indicated substituents, provided that the designated atom's normal valence is not exceeded, and that the substitution results in a stable compound, *i.e*., a compound that can be isolated and characterized using conventional means. For example, substitution can be in the form of an oxygen bound to any other chemical atom than carbon, e.g. hydroxyl group, or an oxygen anion. When a substituent is oxo, *i.e*., =O, then 2 hydrogens on the atom are replaced. An oxo group that is a substituent of an aromatic carbon atom results in a conversion of -CH- to -C(=O)- and a loss of aromaticity. For example, a pyridyl group substituted by oxo is a pyridone.

The term "heteroatom" as used herein shall be understood to mean atoms other than carbon and hydrogen such as and preferably O, N, S and P.

In the context of the present invention it is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₀) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. "(C₁₋₁₀)alkyl" denotes a hydrocarbon residue containing from 1 to 10 carbon atoms, e.g. a methyl, ethyl, propyl, *iso*-propyl, *n*-butyl, *iso*-butyl, *sec*-butyl, *tert*-butyl, *n*-pentyl, *iso*-pentyl, *n*-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C₂₋₁₀)alkenyl" denotes a hydrocarbon residue containing from 2 to 10 carbon atoms, for example an ethenyl (vinyl), propenyl (allyl), *iso*-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH₂]-, as, for example, found in polyunsaturated fatty acids. Alkenyl groups have one or more, preferably 1, 2, 3, 4, 5, or 6 double bond(s).

The expression "alkynyl" refers to at least partially unsaturated, substituted or non-substituted straight-chain or branched hydrocarbon groups that contain the number of carbon items indicated, e.g. "(C₂₋₁₀)alkynyl" denotes a hydrocarbon residue containing from 2 to 10 carbon atoms, for example an ethinyl, propinyl, butinyl, acetylenyl, or propargyl group. Preferably, alkynyl groups have one or two (especially preferably one) triple bond(s).

Furthermore, the terms "alkyl", "alkenyl" and "alkynyl" also refer to groups in which one or more hydrogen atom(s) have been replaced, e.g. by a halogen atom, preferably F or Cl, such as, for example, a 2,2,2-trichloroethyl or a trifluoromethyl group.

The term "carbocycle" shall be understood to mean a substituted or non-substituted aliphatic hydrocarbon cycle containing the number of carbon items indicated, e.g. "(C₃₋₁₀)-carbocycle" or from 3 to 20, preferably from 3 to 12 carbon atoms, more preferably 5 or 6 carbon atoms. These carbocycles may be either aromatic or non-aromatic systems. The non-aromatic ring systems may be mono- or polyunsaturated.

The term "carbobicycle" refers to a carbocycle as defined above comprising more than 1 ring, preferably two rings. Preferred carbocycles and carbobicycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl, benzocycloheptenyl, spiro[4,5]decanyl, norbornyl, decalinyl, bicyclo[4.3.0]nonyl, tetraline, or cyclopentylcyclohexyl. The carbo- and/or carbobicyclic residue may be bound to the remaining structure of the complete molecule by any atom of the cycle, which results in a stable structure

The term "carbocycle" shall also include "cycloalkyl" which is to be understood to mean aliphatic hydrocarbon-containing rings preferably having from 3 to 12 carbon atoms. These non-aromatic ring systems may be mono- or polyunsaturated, i.e. the term encompasses cycloalkenyl and cycloalkynyl.

The term "heterocycle" refers to a stable substituted or non-substituted, aromatic or non-aromatic, preferably 3 to 20 membered, more preferably 3 - 12 membered, most preferably 5 or 6 membered, monocyclic, heteroatom-containing cycle. Each heterocycle consists of carbon atoms and one or more, preferably 1 to 4, more preferably 1 to 3 heteroatoms preferably chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

The term "heterobicycle" refers to a heterocycle as defined above comprising more than 1 ring, preferably two rings.

The hetero- and/or heterobicyclic residue may be bound to the remaining structure of the complete molecule by any atom of the cycle, which results in a stable structure. Exemplary heterocycles and heterobicycles include, but are not limited to pyrrolidinyl, pyrrolinyl, morpholinyl, thiomorpholinyl, thiomorpholinyl sulfoxide, thiomorpholinyl sulfone, dioxalanyl, piperidinyl, piperazinyl, tetrahydrofuranyl, 1-oxo-λ4-thiomorpholinyl, 13-oxa-11-aza-tricyclo[7.3.1.0-2,7]-tridecy-2,4,6-triene, tetrahydropyranyl, 2-oxo-2H-pyranyl, tetrahydrofuranyl, 1,3-dioxolanone, 1,3-dioxanone, 1,4-dioxanyl, 8-oxa-3-aza-bicyclo[3.2.1]octanyl, 2-oxa-5-aza-bicyclo[2.2.1]heptanyl, 2-thia-5-aza-bicyclo[2.2.1]heptanyl, piperidinonyl, tetrahydro-pyrimidonyl, pentamethylene sulphide, pentamethylene sulfoxide, pentamethylene sulfone, tetramethylene sulphide, tetramethylene sulfoxide and tetramethylene sulfone, indazolyl, benzimidazolyl, benzodioxolyl, imidazolyl, 1,3-benzodioxolyl and pyrazolyl.

The expressions "alkyl/alkenyl/alkynyl ether" refer to a saturated or non-saturated, straight-chain or branched hydrocarbon group that contains the number of carbon items indicated. For example, "(C₂₋₁₀)alkyl ether" denotes a hydrocarbon residue containing from 2 to 10 carbon atoms, and any suitable number of oxygen atoms that will result in an ether structure. Alkyl/alkenyl/alkynyl ether groups as used herein shall be understood to mean any linear or branched, substituted or non-substituted alkyl/alkenyl/alkynyl chain comprising an oxygen atom either as an ether motif, i.e. an oxygen bound by two carbons. The ether residue can be attached to the Formulas provided in the present invention either via the carbon atom or via the oxygen atom of the ether residue.

The "substituent" or "residue" or "**R**" as used herein, preferably **R¹**, **R²**, **R³**, **R⁴**, **R⁵**, **R⁶**, **R⁷**, and/or **R⁸**, unless specifically noted otherwise, can be attached directly to the Formulas provided in the present invention or by means of a linker. Said linker can also be in the form of polyethyleneglycol. The term polyethyleneglycol as used herein refers to a chain of substituted or non-substituted ethylene oxide monomers.

As used herein, the terms "nitrogen" or "N" and "sulphur" or "S" include any oxidized form of nitrogen and sulphur and the quaternized form of any basic nitrogen as long as the resulting compound is chemically stable. For example, for an -S-C₁₋₆ alkyl radical shall be understood to include -S(O)-C₁₋₆ alkyl and -S(O)₂-C₁₋₆ alkyl.

As used herein, a wording defining the limits of a range of length such as, e. g., "from 1 to 5" or "(C₁₋₅)" means any integer from 1 to 5, i. e. 1, 2, 3, 4 and 5. In other words, any range defined by two integers explicitly mentioned is meant to comprise and disclose any integer defining said limits and any integer comprised in said range.

By way of example, the term "mono- or di-substituted in *meta* position or mono-substituted in *para* position", as used herein, means that a compound is either substituted by at least one given substituent in *para* position to the position where the compound is attached to another compound or residue, or substituted in two of its *meta* positions by at least one substituent. For example, the term "di-substituted in *meta* position by (C₃)carbocycle or -(CF₃)" denotes that a compound is substituted by one (C₃)carbocycle or -(CF₃) in each *meta* position or by a (C₃)carbocycle in one *meta* position and by -(CF₃) in the other *meta* position. Preferably, the term denotes that a compound is substituted by one (C₃)carbocycle in each *meta* position or by one -(CF₃) in each *meta* position, i.e. is substituted in both *meta* positions by the same substituent. As denoted above for the *para* position, the *meta* position denotes the position *meta* to the position where the compound is attached to another compound or residue.

The compounds as described herein and in the claims include compounds that, e.g. for reasons of metabolic stability, feature the exchange of one or more carbon-bonded hydrogens, preferably one or more aromatic carbon-bonded hydrogens, with halogen atoms such as F, Cl, or Br, preferably F.

In a further aspect, the present invention is directed to a retroaldolase selected from the group consisting of
(a) a retroaldolase comprising, preferably having, an amino acid sequence having an amino acid sequence identity of at least 70% or 80%, preferably at least 90 or 95%., most preferably 95% or 98% with SEQ ID NO: 3,
   with the *proviso* that the retroaldolase does not comprise one of sequences SEQ ID NO:1 and SEQ ID NO: 2;
(b) a retroaldolase comprising functional fragments and/or functional derivatives of (a); and
(c) a retroaldolase according to (a) or (b), wherein in SEQ ID NO: 3 or 4, position position 50 is tyrosine, position 82 is lysine and position 109 is asparagine and/or position 179 is preferably tyrosine, more preferably phenylalanine,
wherein the retroaldolase of (a), (b) and (c) catalyzes the preparation of tertiary alcohols, preferably chiral tertiary alcohols, by an aldol reaction.

All definitions and explanations provided above in the context of the methods and uses of the present invention also apply to the retroaldolase as such according the present invention.

In a preferred embodiment, the retroaldolase of the present invention also catalyzes a reaction selected from the group consisting of
(i) chiral resolution of tertiary alcohols by retroaldol cleavage; and
(ii) deuteration or tritiation of carbonyl compounds, preferably at the α-position of the carbonyl group of the carbonyl compounds, more preferably regio- and/or stereoselective deuteration or tritiation of carbonyl compounds, most preferably with the *proviso* that the carbonyl compounds are not acetone.

In another preferred embodiment, the retroaldolase of the present invention is modified in one or more of positions 11, 111, 132, 183 and 209 of SEQ ID NO: 3, preferably is modified in position 11 by glycine, phenylalanine or alanine; in position 111 by isoleucine, leucine or valine; in position 132 by phenylalanine; in position 183 by valine or tyrosine; in position 209 by isoleucine or alanine; and/or combinations thereof.

In a further preferred embodiment, the retroaldolase of the present invention is selected from the group consisting of
(a) a retroaldolase comprising, preferably having, an amino acid sequence according to SEQ ID NOs: 5 to 28;
(b) a retroaldolase comprising, preferably having, an amino acid sequence having an amino acid sequence identity of at least 70% or 80%, preferably at least 90 or 95%., most preferably 95% or 98% with SEQ ID NOs: 5 to 28,
   with the *proviso* that the retroaldolase does not comprise one of sequences SEQ ID NO:1 and SEQ ID NO: 2; and
(c) a retroaldolase comprising functional fragments and/or functional derivatives of any of (a) and/or (b).

In a further preferred embodiment, the retroaldolase of the present invention catalyzes the production of the tertiary alcohols or the deuterated or tritiated carbonyl compounds stereospecifically, more preferably diastereospecifically and/or enantiospecifically.

In another preferred embodiment, the retroaldolase of the present invention catalyzes the production of the tertiary alcohols with a diastereomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and/or an enantiomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and the deuterated or tritiated carbonyl compounds with a diastereomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and/or an enantiomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9.

In another preferred embodiment, the retroaldolase of the present invention catalyzes the reaction 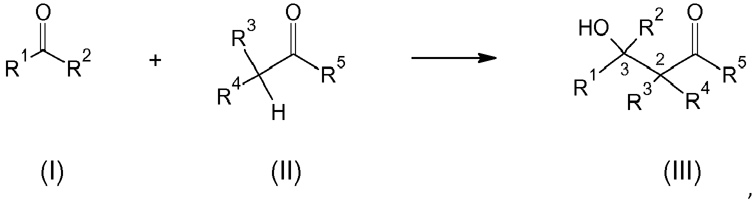
wherein at least one of R¹ or R² is an electron withdrawing residue, and
**R¹** and **R²** are independently selected from the group consisting of
   (i) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether, wherein the ether is bonded to formula (I) via its carbon atom;
   (ii) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
   (iii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl;
   (iv) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S; and
   (v) an electron withdrawing group, preferably selected from the group consisting of -COOR⁶, - CR⁷_{d}, -S(O)₂OH, -CONR¹R², wherein
      (aa) **R⁶** is selected from the group consisting of hydrogen, R¹, preferably substituted or non-substituted methyl, ethyl and propyl, most preferably methyl and ethyl;
      (bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein R¹ and/or R² are bonded directly to formula (I), via -O-, or via a -(CHₐ)_{b}- linker, wherein **a** is an integer from 0 to 2 and **b** is an integer from 1 to 10; preferably R¹ and/or R² are selected from the group consisting of -COOH, -COOMe, -COOEt, -CF₃, CHF₂, -CCl₃, and/or are bonded directly to Formula (I);
**R³** and **R⁴** are independently selected from the group consisting of
   (i) hydrogen, F, Cl, Br, R⁸, N(R⁸)e, OR⁸, S(R⁸), P(R⁸)_{f} and C(R⁸)_{d}, wherein **e** is 1 or 2, **f** is an integer from 1 to 4, **d** is an integer from 1 to 3, and **R⁸** is independently selected from the group consisting of
      (aa) hydrogen, F, Cl, Br, NO₂ and oxo;
      (bb) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether;
      (cc) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
      (dd) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl; and
      (ee) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S; and
   (ii) an electron withdrawing group, preferably selected from the group consisting of -COOR⁶, - CR⁷_{d}, -S(O)₂OH, -CONR¹R², wherein
      (cc) **R⁶** is selected from the group consisting of hydrogen, R¹, preferably substituted or non-substituted methyl, ethyl and propyl, most preferably methyl and ethyl;
      (dd) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein the electron withdrawing group is bonded directly to formula (II), via -O-, or via a - (CHₐ)_{b}- linker, wherein **a** is an integer from 0 to 2 and **b** is an integer from 1 to 10; preferably the electron withdrawing group is selected from -COOH, -COOMe, -COOEt, -CF₃, CHF₂, -CCl₃, and/or is bonded directly to Formula (II);
**R⁵** is hydrogen or C(R⁸)_{d}, wherein **d** is an integer from 1 to 3, and **R⁸** is as defined above;
wherein the tertiary alcohol (III) is preferably a chiral tertiary alcohol and the stereogenic carbon atoms (2) and (3) of tertiary alcohol (III) are (*R,R*)*-,* (*S,R*)*-,* (*R,S*)- or (*S,S*)-configured, preferably with a diastereomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9; and/or an enantiomeric ratio selected from the group consisting of greater than 1:1, 1:1.5 to 1:9, 1:2 to 1:9, 1:3 to 1:9, 1:5 to 1:9, 1:7 to 1:9 and 1:8 to 1:9.

Preferred substrates for the chiral resolution of tertiary alcohols by retroaldol cleavage catalyzed by the retroaldolase of the present invention, are substrates as defined in Formula (III) above. It is further preferred, that residues R¹ and/or R² of these substrates according to Formula (III) are selected from groups (i) to (iv), i.e. are not electron withdrawing groups.

Preferred substrates for the deuteration or tritiation of carbonyl compounds catalyzed by the retroaldolase of the present invention, are substrates as defined in Formula (II), wherein R³ and/or R⁴ are not hydrogen.

In another preferred embodiment, the retroaldolase of the present invention catalyzes the preparation of the tertiary alcohols without the step of decarboxylation and/or the preparation of cyanides.

The following Figures and Examples serve to illustrate the invention and are not intended to limit the scope of the invention as described in the appended claims.
**Figure 1****:** shows a HPLC chromatogram for the analysis of the product of Example 1 (ethyl (*S*)-2-hydroxy-4-oxo-2-phenethylpentanoate) compared to the racemic aldol reaction product under the following conditions: Chiralcel OD-H, n-hexane / i-PrOH = 9 : 1, flow rate 0.6 mL/ min, λ = 210 nm.
**Figure 2****:** shows a HPLC chromatogram for the analysis of the resolution product of Example 3 under the following conditions: Chiralcel OD-H, n-hexane / i-PrOH = 9 : 1, flow rate 0.6 mL / min, λ = 230 nm.
**Figure 3****:** shows the 1H-NMR comparison of the reaction products of Example 4.
**Figure 4A****-B:** is a schematic representation of the active site of RA95.5-8F (SEQ ID NO: 1). Lysine 83 is covalently bonded to the substrate analog 1-(6-methoxynaphthalen-2-yl)butane-1,3-dione, shown in dark grey. Figure 4 **A** highlights residues important for catalysis, and Figure 4 **B** shows residues important for stereoselectivity and substrate recognition (the figure was created using the program Pymol (Schrödinger, Cambridge, MA, USA) and the PDB access code 5AN7).

### Example 1: Synthesis of (S)-2-hydroxy-4-oxo-2-phenethylpentanoate

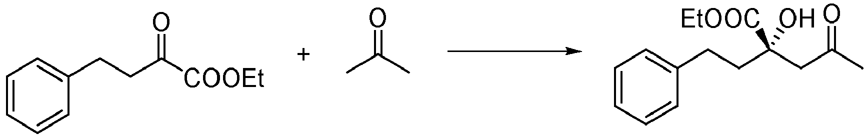

In a 100 mL Erlenmeyer flask, an acetone solution (2 M final concentration) containing ethyl 2-oxo-4-phenylbutanoate (0.12 mmol, 24.75 mg) was mixed with buffer solution (25 mM HEPES, 100 mM NaCl, pH 7.5) containing RA95.5-8F (SEQ ID NO: 1, final concentration 1µM) and the reaction mixture was incubated at 29 ºC 600 rpm in an orbital shaker. Reaction progress was monitored by HPLC, and conversion quantified using solutions of the purified aldol adduct as external standard. After 24h reaction time, the reaction mixture was saturated with NaCl, extracted with EtOAc, and the organic phase dried over sodium sulfate. Solvent was removed under vacuum. The crude material was purified by flash chromatography (EtAcO/Hexane 1:2) to obtain ethyl (*S*)-2-hydroxy-4-oxo-2-phenethylpentanoate (21.87 mg, 70.1% isolated yield) as a colorless oil. The stereochemistry of the product (*R*/*S* = 0.4 : 99.6) was established by chiral HPLC by comparison with published data (Tetrahedron Lett. 2010, 51, 1884-1886): Chiralcel OD-H, n-hexane / i-PrOH = 9 : 1, flow rate 0.6 mL/min, λ = 210 nm.

### Example 2: Stereoselective aldol reactions

The following reactions were performed with the catalysts listed in Table 1 below.

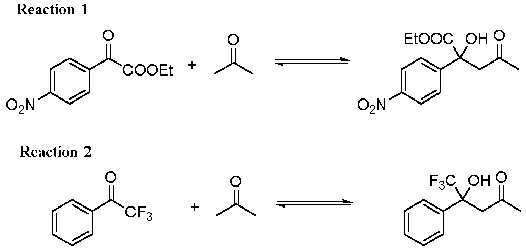

### Reaction 1:

Reaction conditions: 2.0 M acetone, 5.0 mM ethyl 2-(4-nitrophenyl)-2-oxoacetate; analysis by chiral HPLC analysis: Chiralcel OD-H column, λ = 210 nm, i-PrOH:Hexane = 10:90, 0.6 mL/min

### Reaction 2:

Reaction conditions: 2.0 M acetone, 5.0 mM 2,2,2-trifluoro-1-phenylethan-1-one; analysis by chiral HPLC analysis: Chiralcel OD-H column, λ = 210 nm, i-PrOH:Hexane = 3:97, 1.0 mL/min

**Table 1: Catalysts used in reactions 1 and 2 above**

| **Catalyst** | **Reaction 1 Selectivity (*R*/*S*)** | **Reaction 2 Selectivity (*R*/*S*)** |
|---|---|---|
| RA95.5-8F (SEQ ID NO: 1) | 51.8:48.2 | 68.9:31.1 |
| RA95.5-8F F112I (SEQ ID NO: 30) | 85.4:4.6 | 63.2:36.8 |
| RA95.5-8F F112L (SEQ ID NO: 31) | 79.5:20.5 | 73.1:26.9 |
| RA95.5-8F F112V (SEQ ID NO: 32) | 85.3:14.7 | 67.0:31.0 |
| RA95.5-8F F184V (SEQ ID NO: 33) | 37.4:62.6 | 40.9:59.1 |
| RA95.5-8F L210A (SEQ ID NO: 53) | 60.9:39.1 | 75.9:24.1 |
| RA95.5-8F L210I (SEQ ID NO: 52) | 56.9:43.1 | 62.0:38.0 |
| RA95.5-8F I133F (SEQ ID NO: 34) | 72.4:27:6 | 68.8:31.2 |
| RA95.5-8F V12G F112I (SEQ ID NO: 35) | 88.8:11.2 | 63.5:36:6 |
| RA95.5-8F V12F F112I (SEQ ID NO: 36) | 52.8:47.2 | 70.3:29.7 |
| RA95.5-8F V12I F112L (SEQ ID NO: 37) | 53.5:46.5 | 81.0:19.0 |
| RA95.5-8F V12A F112V (SEQ ID NO: 38) | 90.5:9.5 | 69.5:30.5 |

### Example 3: Resolution of tertiary chiral alcohols:

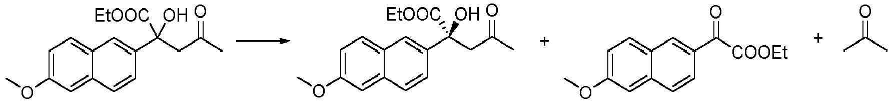

In a 2.0 mL centrifuge tube, an acetonitrile solution (27 µL) containing rac-ethyl 2-hydroxy-2-(6-methoxynaphthalen-2-yl)-4-oxopentanoate (95 µg, final concentration 300 µM) was mixed with buffer solution (973 µL; 25 mM HEPES, 100 mM NaCl, pH 7.5) containing F112I/L210A RA95.5-8F (SEQ ID NO: 50, final concentration 3 µM) and the reaction mixture was incubated at 29 ºC. Aliquots taken at 0 and 48 h reaction time were analyzed by chiral HPLC: Chiralcel OD-H, n-hexane / i-PrOH = 9 : 1, flow rate 0.6 mL / min, λ = 230 nm. Selective cleavage of one of the two enantiomers was observed, with a final enantiomeric ratio of 2:98.

### Example 4: Deuteration of cyclohexanone

Buffered solutions (989.6 µL; HEPES 25mM, NaCl 100mM, pH = 7.5) containing no catalyst (A), RA95.5-8F (SEQ ID NO: 1, 2 µM; B), and F112I RA95.5-8F (SEQ ID NO: 30, 2 µM; C) were dried by lyophilization in 2.0 mL centrifuge tubes and the resulting solid was subsequently resuspend-ded in D₂O (989.6 µL; 99.90 % purity). Cyclohexanone (10.4 µL, 100 mM final conc.) was added and the reactions were incubated at 29 °C. After 2 h reaction time, the reactions were extracted with deuterated chloroform (1.0 mL). The organic phases were dried with Na₂SO₄, centrifuged, and analyzed by ¹H-NMR. Significant deuteration is not detected in absence of catalyst or using RA95.5-8F. In contrast, in presence of F112I RA95.5-8F (SEQ ID NO: 30), single deuteration of carbon-2 and carbon-6 of cyclohexanone (i.e. the positions in alpha of the carbonyl function) is observed, thus yielding cyclohexan-1-one-2,6-*d*₂.

### Example 5: Protein expression

RA95.5-8 (SEQ ID NO: 2), RA95.5-8F (SEQ ID NO: 1) and its variants (SEQ ID NOs: 30 - 53) were subcloned into the commercial pET-29b(+) vector (Novagen). Individual variants were produced as C-terminally His-tagged proteins in *E. coli* BL21-Gold(DE3) and purified by affinity chromatography. To ensure monoclonality, single-colony streakouts of the most active clones were prepared from the library master plates. Single colonies were used to prepare precultures, of which 0.5 mL were inoculated in 500 mL of LB medium containing 30 µg mL⁻¹ kanamycin sulfate. The bacterial cultures were incubated at 37 °C and 220 rpm until a D_{600 nm} of 0.4 was reached. Following induction of enzyme production with 0.1 mM IPTG, the cells were incubated for an additional 5 h at 37 °C, at which point they were harvested. Cell pellets were stored at -20 °C before lysis. Upon thawing, the pellets were resuspended in 25 mM HEPES, 300 mM NaCl, pH 7.5, containing 1 mg mL⁻¹ egg white lysozyme and incubated for 1 h at 4 °C. The cells were then lysed by sonication, and cell debris was removed by centrifugation. Ni-NTA beads (Qiagen, Venlo, Netherlands) were equilibrated with 25 mM HEPES, 300 mM NaCl, pH 7.5, and the soluble protein fraction was loaded onto the column. The samples were washed once with the same buffer without imidazole and subsequently with buffer containing 20 mM and 40 mM imidazole. The protein was finally eluted using 200 mM imidazole. The sample was dialyzed at 4 °C against 25 mM HEPES, 100 mM NaCl, pH 7.5. Protein concentration was determined from the absorbance at 280 nm using calculated extinction coefficients (Gasteiger E., et al., The Proteomics Protocols Handbook, Humana Press (2005), pp. 571-607).

### Example 6: Expression of aldolase libraries

Plasmids were transformed in BL21-Gold (DE3) cells, plated on Petri plates (LB media, 30 mg·L¹ kanamycin) and incubated overnight at 37 ºC. Ninety six-well micro titer plates, containing 150 µL of LB media with 30 mg·L¹ kanamycin per well, were inoculated with single colonies using sterile tooth picks. Two wells were inoculated with clean toothpicks as blank controls, and four wells were inoculated with a single colony of RA95.5-8F (SEQ ID NO: 1) as a reference of 100% of activity. The plates were covered with air permeable membranes (Breathe Easy, Diversified Biotech) and incubated overnight at 30 ºC and 900 rpm Pre-warmed (37 ºC) 96 deep-well plates (Deepwell plate 96/2000 µL, Eppendorf) containing 1.5 mL LB-medium (30 mg·L¹ kanamycin) per well were inoculated with the pre-culture (24 µL per well), covered with an air permeable membrane (Breathe Easy, Diversified Biotech), and incubated at 37 ºC and 600 rpm After 135 min, protein expression was induced with an IPTG solution (30 µL 5 mM, final concentration 0.1 mM), and the plates were incubated for additional 5 h at 37 ºC and 600 rpm The cells were harvested by centrifugation (4000 rpm, 4 ºC, 15 min) and the supernatant was completely discarded. The pellets were suspended in 400 µL of assay buffer (25 mM HEPES 100 mM NaCl, pH 7.5) supplemented with 1 mg·mL⁻¹ lysozyme from chicken egg. The plate was incubated (600 rpm, room temperature) for 1 h and stored overnight at -20 ºC. The plates were thawed and incubated (600 rpm, room temperature) for 1 h and the suspensions were cleared by centrifugation (4000 rpm, 20 °C, 20 min).

### Example 7: Spectroscopic analysis of aldolase libraries

For the assay, 20 µL of the cleared lysates were transferred to a 96-well microtiter plate containing in each well 174 µL of assay buffer (25 mM HEPES 100 mM NaCl, pH 7.5) and 6 µL of an acetonitrile solution of enantiopure or racemic ethyl 2-hydroxy-2-(6-methoxynaphthalen-2-yl)-4-oxopentanoate (0.03 µmol, 9.5 µg, final concentration 150 µM), or an alternative aldol adduct resulting of the addition of a nucleophile ketone of interest to ethyl 2-(6-methoxynaphthalen-2-yl)-2-oxoacetate. Enzymatic activity was measured in a UV plate reader (Thermofisher Scientific Varioscan) monitoring the absorbance decrease at 350 nm.

### Example 8: Chromatographic analysis of aldolase libraries

For the assay, 20-100 µL of the cleared lysates were transferred to a 96-well polypropylene multi-well plate (Deepwell plate 96/2000 µL, Eppendorf) containing in each well 180-100 µL of a buffered solution (25 mM HEPES 100 mM NaCl, pH 7.5) with the nucleophilic ketone (5-3000 mM final concentration) and the electrophilic ketone (1-50 mM). The plate was incubated for 48 h at 20-29 ºC. Conversion was determined by HPLC analysis of reaction samples (20 µL) diluted in acetonitrile (180 µL). The crude reactions of active variants (i.e. yielding >30 % conversion) were extracted by addition of 300 µL methyl *tert*-butyl ether and vigorous shaking for 2 min. After centrifugation (3 min, 12 ºC, 2500 r.c.f.) a fraction of the organic phase (200 µL) was transferred to a fresh multi-well plate (MicroWell, Nunc). The solvent was evaporated under a flow of air subsequently under reduced pressure (1-2 mbar) for 10 min. The crude products were resuspended in 150 µL of heptane/isopropanol mixture of variable composition. The solutions were transferred to a 96-well filter plate (0.2 µm pore-size PTFE membranes, AcroPrep, Pall Corporation) and centrifuged (2 min, 12 ºC, 2500 r.c.f.) into 96-well polypropylene plates (MicroWell, Nunc). The filtered solutions were transferred into glass vials and 30 µL samples were injected and analyzed by chiral HPLC.

### Example 9: Analysis of purified variants of RA95.5-8F (SEQ ID NO: 1)

**Spectroscopic analysis:** Reactions were carried out at 29 °C in aqueous buffer (25 mM HEPES, 100 mM NaCl, pH 7.5) in 1 mL sealed quartz cuvettes using RA95.5-8F (SEQ ID NO: 1) or its variants (SEQ ID NOs: 30-53) as catalysts. Acetonitrile at a final concentration of 2.7% was included as co-solvent to facilitate substrate solubility. The retro-aldol cleavage of rac-ethyl 2-hydroxy-2-(6-methoxynaphthalen-2-yl)-4-oxopentanoate to give ethyl 2-(6-methoxynaphthalen-2-yl)-2-oxoacetate and acetone was monitored spectroscopically at 350 nm (Δε = 8641 M⁻¹cm⁻¹) using a Perkin Elmer Lambda 35 UV-vis spectrometer equipped with a Peltier system for temperature control. The data were corrected for the buffer-catalyzed background reaction measured under the same conditions. Steady-state kinetic parameters were derived by fitting the experimental data to the Michaelis-Menten equation: *v*₀/[E] = *k*_{cat} · [S]/(*K*_{M} + [S]), where *v*₀ is the initial rate, [E] is the enzyme concentration, K_{M} is the Michaelis constant, and [S] is the substrate concentration.

**Chromatographic analysis:** Reactions were conducted in 1.5 mL centrifuge tubes incubated in a water bath thermostated at 29 ºC. The electrophilic ketone (1-50 mM final concentration) and the nucleophilic ketone (5-3000 mM final concentration) were mixed, and the enzyme solution (0.05-1.0 nmol, 0.1-10µM final concentration) in buffer (sufficient amount for 500uL total volume, 25 mM HEPES 100 mM NaCl, pH = 7.5) was added. Conversions were determined at 3 h and 24 h reaction time. Reaction monitoring was as follows: aliquots (20 µL) were withdrawn, diluted with acetonitrile (120 µL) and analyzed by HPLC. The reaction crudes were subsequently extracted by addition of 600 µL methyl *tert*-butyl ether and vigorous shaking for 2 min. After centrifugation (3 min, 12 ºC, 2500 r.c.f.) a fraction of the organic phase (400 µL) was transferred to a fresh multi-well plate (MicroWell, Nunc). The solvent was evaporated under a flow of air subsequently under reduced pressure (1-2 mbar) for 10 min. The crude products were resuspended in 150 µL of heptane/isopropanol mixture of variable composition. The solutions were transferred to a 96-well filter plate (0.2 µm pore-size PTFE membranes, AcroPrep, Pall Corporation) and centrifuged (2 min, 12 ºC, 2500 r.c.f.) into 96-well polypropylene plates (MicroWell, Nunc). The filtered solutions were transferred into glass vials and 30 µL samples were injected and analyzed by chiral HPLC.

## Claims

1. A method for catalyzing a chemical reaction selected from the group consisting of:
(i) preparing tertiary alcohols, preferably chiral tertiary alcohols, by an aldol reaction;
(ii) chiral resolution of tertiary alcohols by retroaldol cleavage; and
(iii) deuteration or tritiation of carbonyl compounds, preferably at the α-position of the carbonyl group of the carbonyl compounds, more preferably regio- and/or stereoselective deuteration or tritiation of carbonyl compounds, most preferably with the *proviso* that the carbonyl compounds are not acetone,
comprising the steps of:
(a) providing a retroaldolase selected from the group consisting of:
(aa) a retroaldolase comprising an amino acid sequence selected from the group consisting of SEQ ID NOs: 3 to 28;
(ab) a retroaldolase comprising an amino acid sequence having an amino acid sequence identity of at least 70% or 80%, preferably at least 90 or 95%, most preferably 95% with an amino acid sequence selected from the group consisting of SEQ ID NOs: 3 to 28;
(ac) a retroaldolase comprising a functional derivative and/or functional fragment of (aa) and/or (ab); and
(ad) a retroaldolase according to any of (aa) to (ac), wherein in SEQ ID NOs: 3 to 28, position 50 is tyrosine, position 82 is lysine and position 109 is asparagine and/or position 179 is preferably tyrosine, more preferably phenylalanine,
preferably the retroaldolase comprising SEQ ID NO: 30, or the retroaldolase comprising SEQ ID NO: 34, a functional derivative and/or functional fragment of these,
for catalyzing the chemical reaction (i), (ii) or (iii),
(b) providing at least one substrate for the chemical reaction (i), (ii) or (iii) selected from the group consisting of
(ba) (baa) an aldehyde-comprising substrate and a ketone-comprising substrate, preferably a nucleophilic aldehyde-comprising substrate and an electrophilic ketone-comprising substrate, or (bab) two ketone-comprising substrates, which substrates react in the aldol reaction (i) to form a tertiary alcohol;
(bb) tertiary alcohols for chiral resolution by retroaldol cleavage (ii); and
(bc) carbonyl compounds, preferably with the *proviso* that the carbonyl compounds are not acetone, for deuteration or tritiation (iii);
(c) contacting the retroaldolase of (a) with the substrate of (b) under conditions that allow enzymatic activity of the retroaldolase and the chemical reaction to proceed, and
(d) optionally purifying the product of the chemical reaction.

2. The method according to claim 1, further comprising the step (f) of modifying the retroaldolase of (a), preferably in at least one of positions 11, 111, 132, 183 and 209 of SEQ ID NO: 3 to 28, wherein step (f) is performed after step (a) and before step (c).

3. A method for modifying a retroaldolase for catalyzing a chemical reaction selected from the group consisting of:
(i) preparing tertiary alcohols, preferably chiral tertiary alcohols, by an aldol reaction;
(ii) chiral resolution of tertiary alcohols by retroaldol cleavage; and
(iii) deuteration or tritiation of carbonyl compounds, preferably at the α-position of the carbonyl group of carbonyl compounds, more preferably regio- and/or stereoselective deuteration or tritiation of carbonyl compounds, most preferably with the *proviso* that the carbonyl compounds are not acetone,
comprising the steps of:
(a) providing a retroaldolase selected from the group consisting of
a. a retroaldolase comprising an amino acid sequence selected from the group consisting of SEQ ID NOs: 3 to 28;
b. a retroaldolase comprising an amino acid sequence having an amino acid sequence identity of at least 70% or 80%, preferably at least 90 or 95%, most preferably 95% with an amino acid sequence selected from the group consisting of SEQ ID NOs: 3 to 28;
c. a retroaldolase comprising a functional derivative and/or functional fragment of a. and/or b.; and
d. a retroaldolase according to any of a. to c., wherein in SEQ ID NOs: 3 to 28, position 50 is tyrosine, position 82 is lysine and position 109 is asparagine and/or position 179 is preferably tyrosine, more preferably phenylalanine;
(b) modifying at least one amino acid position, preferably at least one of positions 11, 111, 132, 183 and 209 of SEQ ID NO: 3 to 28 in any one of the above retroaldolases a. to d.;
(c) providing at least one substrate of interest for at least one of the above reactions (i) to (iii), and
(d) contacting the at least one substrate of interest of (c) with at least one of the modified retroaldolases a. to d. under conditions that allow enzymatic activity of the retroaldolase and the reaction to proceed, and
(e) identifying at least one modified retroaldolase that catalyzes, preferably stereospecifically catalyzes at least one of the reactions (i) to (iii).

4. The method according to claim 2 or 3, wherein in step (f) of claim 2 or step (b) of claim 3, the retroaldolase is modified in one or more of the following positions of SEQ ID NO: 3 to 28, preferably in SEQ ID NO: 3: in position 11 by glycine, phenylalanine or alanine; in position 111 by isoleucine, leucine or valine; in position 132 by phenylalanine; in position 183 by valine or tyrosine; and/or in position 209 by isoleucine or alanine.

5. Use of a retroaldolase selected from the group consisting of
(a) a retroaldolase comprising an amino acid sequence selected from the group consisting of SEQ ID NOs: 3 to 28;
(b) a retroaldolase comprising an amino acid sequence having an amino acid sequence identity of at least 70% or 80%, preferably at least 90 or 95%, most preferably 95% with an amino acid sequence selected from the group consisting of SEQ ID NOs: 3 to 28; and
(c) a retroaldolase comprising a functional derivative and/or functional fragment of (a) and/or (b),
(d) a retroaldolase according to any of (a) to (c), wherein in SEQ ID NO: 3 to 28, position 50 is tyrosine, position 82 is lysine and position 109 is asparagine and/or position 179 is preferably tyrosine, more preferably phenylalanine,
for catalyzing a chemical reaction selected from the group consisting of:
(i) preparing tertiary alcohols, preferably chiral tertiary alcohols, by an aldol reaction;
(ii) chiral resolution of tertiary alcohols by retroaldol cleavage; and
(iii) deuteration or tritiation of carbonyl compounds, preferably at the α-position of the carbonyl group of the carbonyl compounds, more preferably regio- and/or stereoselective deuteration or tritiation of carbonyl compounds, most preferably with the *proviso* that the carbonyl compounds are not acetone.

6. The use of the retroaldolase according to claim 5, wherein the retroaldolase is modified in one or more of positions 11, 111, 132, 183 and 209 of SEQ ID NO: 3 to 28, preferably of SEQ ID NO: 3, preferably is modified in position 11 by glycine, phenylalanine or alanine; in position 111 by isoleucine, leucine or valine; in position 132 by phenylalanine; in position 183 by valine or tyrosine; in position 209 by isoleucine or alanine; and/or combinations thereof.

7. The method according to any of claims 1 to 4 or the use according to any of claims 5 to 6, wherein the aldol reaction and/or the deuteration or tritiation reaction is a stereospecific reaction, preferably a diastereospecific and/or enantiospecific reaction.

8. The method according to any of claims 1 to 4 or 7, or the use according to any of claims 5 to 7, wherein the retroaldolase catalyzes the reaction 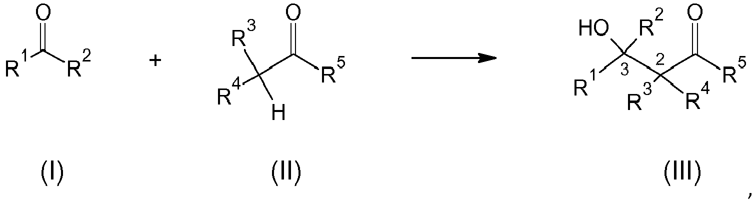
wherein at least one of R¹ or R² is an electron withdrawing residue, and
**R¹** and **R²** are independently selected from the group consisting of
(i) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether, wherein the ether is bonded to formula (I) via its carbon atom;
(ii) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
(iii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl;
(iv) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S; and
(v) an electron withdrawing group, preferably selected from the group consisting of - COOR⁶, -CR⁷_{d}, -S(O)₂OH, -CONR¹R², wherein
(aa) **R⁶** is selected from the group consisting of hydrogen, R¹, preferably substituted or non-substituted methyl, ethyl and propyl, most preferably methyl and ethyl;
(bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein R¹ and/or R² are bonded directly to formula (I), via -O-, or via a -(CHₐ)_{b}- linker,
wherein a is an integer from 0 to 2 and **b** is an integer from 1 to 10; preferably R¹ and/or R² are selected from the group consisting of -COOH, -COOMe, -COOEt, -CF₃, CHF₂, -CCl₃, and/or are bonded directly to Formula (I);
**R³** and **R⁴** are independently selected from the group consisting of
(i) hydrogen, F, Cl, Br, R⁸, N(R⁸)ₑ, OR⁸, S(R⁸), P(R⁸)_{f} and C(R⁸)_{d}, wherein e is 1 or 2, f is an integer from 1 to 4, **d** is an integer from 1 to 3, and **R⁸** is independently selected from the group consisting of
(aa) hydrogen, F, Cl, Br, NO₂ and oxo;
(bb) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether;
(cc) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
(dd) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl; and
(ee) substituted or non-substituted (C₃₋₆)heterocycle and (C₇-_{C10})carbo- or hetero-bicycle having 1 to 3 heteroatoms each independently selected from N, O and S, preferably substituted or non-substituted (C₇)heterobicycle having 2 heteroatoms selected from N and S; and
(ii) an electron withdrawing group, preferably selected from the group consisting of - COOR⁶, -CR⁷_{d}, -S(O)₂OH, -CONR¹R², wherein
(aa) **R⁶** is selected from the group consisting of hydrogen, R¹, preferably substituted or non-substituted methyl, ethyl and propyl, most preferably methyl and ethyl;
(bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein the electron withdrawing group is bonded directly to formula (II), via -O-, or via a -(CHₐ)_{b}- linker, wherein **a** is an integer from 0 to 2 and **b** is an integer from 1 to 10;
preferably the electron withdrawing group is selected from -COOH, -COOMe, -COOEt, -CF₃, CHF₂, -CCl₃, and/or is bonded directly to Formula (II);
**R⁵** is hydrogen or C(R⁸)_{d}, wherein **d** is an integer from 1 to 3, and **R⁸** is as defined above; wherein the tertiary alcohol (III) is preferably a chiral tertiary alcohol and the stereogenic carbon atoms (2) and (3) of tertiary alcohol (III) are (*R,R*)*-,* (*S,R*)*-,* (*R,S*)- or (*S,S*)-configured.

9. A retroaldolase selected from the group consisting of
(a) a retroaldolase comprising an amino acid sequence having an amino acid sequence identity of at least 70% or 80%, preferably at least 90 or 95%., most preferably 95% with SEQ ID NO: 3,
with the *proviso* that the retroaldolase does not comprise one of sequences SEQ ID NO:1 and SEQ ID NO: 2;
(b) a retroaldolase comprising functional fragments and/or functional derivatives of (a); and
(c) a retroaldolase according to (a) or (b), wherein in SEQ ID NO: 3 or 4, position position 50 is tyrosine, position 82 is lysine and position 109 is asparagine and/or position 179 is preferably tyrosine, more preferably phenylalanine,
wherein the retroaldolase of (a), (b) and (c) catalyzes the preparation of tertiary alcohols, preferably chiral tertiary alcohols, by an aldol reaction.

10. The retroaldolase according to claim 9, wherein the retroaldolase also catalyzes a reaction selected from the group consisting of
(i) chiral resolution of tertiary alcohols by retroaldol cleavage; and
(ii) deuteration or tritiation of carbonyl compounds, preferably at the α-position of the carbonyl group of the carbonyl compounds, more preferably regio- and/or stereoselective deuteration or tritiation of carbonyl compounds, most preferably with the *proviso* that the carbonyl compounds are not acetone.

11. The retroaldolase according to claim 9 or 10, wherein the retroaldolase is modified in one or more of positions 11, 111, 132, 183 and 209 of SEQ ID NO: 3, preferably is modified in position 11 by glycine, phenylalanine or alanine; in position 111 by isoleucine, leucine or valine; in position 132 by phenylalanine; in position 183 by valine or tyrosine; in position 209 by isoleucine or alanine; and/or combinations thereof.

12. The retroaldolase according to any of claims 9 to 11, wherein the retroaldolase is selected from the group consisting of
(a) a retroaldolase comprising an amino acid sequence according to SEQ ID NOs: 5 to 28;
(b) a retroaldolase comprising an amino acid sequence having an amino acid sequence identity of at least 70% or 80%, preferably at least 90 or 95%., most preferably 95% with SEQ ID NOs: 5 to 28,
with the *proviso* that the retroaldolase does not comprise one of sequences SEQ ID NO:1 and SEQ ID NO: 2; and
(c) a retroaldolase comprising functional fragments and/or functional derivatives of any of (a) and/or (b).

13. The retroaldolase according to any of claims 9 to 12, wherein the retroaldolase catalyzes the production of the tertiary alcohols or the deuterated or tritiated carbonyl compounds stereospecifically, more preferably diastereospecifically and/or enantiospecifically.

14. The retroaldolase according to any of claims 9 to 13, wherein the retroaldolase catalyzes the reaction 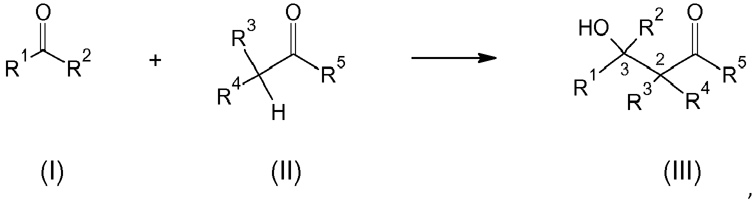
wherein at least one of R¹ or R² is an electron withdrawing residue, and
**R¹** and **R²** are independently selected from the group consisting of
(i) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether, wherein the ether is bonded to formula (I) via its carbon atom;
(ii) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
(iii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl;
(iv) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S;
(v) an electron withdrawing group, preferably selected from the group consisting of-COOR⁶, -CR⁷_{d}, -S(O)₂OH, -CONR¹R², wherein
(aa) **R⁶** is selected from the group consisting of hydrogen, R¹, preferably substituted or non-substituted methyl, ethyl and propyl, most preferably methyl and ethyl;
(bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein R¹ and/or R² are bonded directly to formula (I), via -O-, or via a -(CHₐ)_{b}- linker,
wherein **a** is an integer from 0 to 2 and **b** is an integer from 1 to 10; preferably R¹ and/or R² are selected from the group consisting of -COOH, -COOMe, -COOEt, -CF₃, CHF₂, -CCl₃, and/or are bonded directly to Formula (I);
**R³** and **R⁴** are independently selected from the group consisting of
(i) hydrogen, F, Cl, Br, R⁸, N(R⁸)ₑ, OR⁸, S(R⁸), P(R⁸)_{f} and C(R⁸)_{d}, wherein **e** is 1 or 2, **f** is an integer from 1 to 4, **d** is an integer from 1 to 3, and **R⁸** is independently selected from the group consisting of
(aa) hydrogen, F, Cl, Br, NO₂, and oxo;
(bb) linear or branched, substituted or non-substituted (C₂₋₂₀)alkyl ether, (C₃₋₂₀)alkenyl ether, (C₃₋₂₀)alkynyl ether and (C₄₋₂₀)carbocyclic ether;
(cc) linear or branched, substituted or non-substituted (C₁₋₂₀)alkyl, (C₂₋₂₀)alkenyl, (C₂₋₂₀)alkynyl, preferably (C₁₋₁₀)alkyl, more preferably substituted or non-substituted methyl, ethyl and propyl, most preferably substituted or non-substituted methyl;
(dd) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl and a para-substituted phenyl that is substituted by a substituent selected from the group consisting of Cl, F, Br, substituted or non-substituted methyl, preferably -(CF₃), ethyl, propyl and cyclopropyl; and
(ee) substituted or non-substituted (C₃₋₆)heterocycle and (C_{7-C10})carbo- or hetero-bicycle having 1 to 3 heteroatoms each independently selected from N, O and S, preferably substituted or non-substituted (C₇)heterobicycle having 2 heteroatoms selected from N and S; and
(ii) an electron withdrawing group, preferably selected from the group consisting of-COOR⁶, -CR⁷_{d}, -S(O)₂OH, -CONR¹R², wherein
(aa) **R⁶** is selected from the group consisting of hydrogen, R¹, preferably substituted or non-substituted methyl, ethyl and propyl, most preferably methyl and ethyl;
(bb) **R⁷** is selected from the group consisting of hydrogen, halogens, preferably F, Cl and Br, wherein at least one of R⁷ is a halogen and the remaining residues are hydrogen, and wherein **d** is an integer from 1 to 3;
wherein the electron withdrawing group is bonded directly to formula (II), via -O-, or via a -(CHₐ)_{b}- linker, wherein **a** is an integer from 0 to 2 and **b** is an integer from 1 to 10;
preferably the electron withdrawing group is selected from -COOH, -COOMe, -COOEt, -CF₃, CHF₂, -CCl₃, and/or is bonded directly to Formula (II);
**R⁵** is hydrogen or C(R⁸)_{d}, wherein **d** is an integer from 1 to 3, and **R⁸** is as defined above; wherein the tertiary alcohol (III) is preferably a chiral tertiary alcohol and the stereogenic carbon atoms (2) and (3) of tertiary alcohol (III) are (*R,R*)-, (*S,R*)-, (*R,S*)- or (*S,S*)-configured.

15. The retroaldolase according to any of claims 9 to 14, wherein the retroaldolase catalyzes the preparation of the tertiary alcohols without the step of decarboxylation and/or the preparation of cyanides.
